(19) Europäisches Patentamt
European Patent Office
Office européen des brevets

(11) EP 4 185 652 B1

(12) FASCICULE DE BREVET EUROPEEN

(45) Date de publication et mention de la délivrance du brevet:
**03.06.2026 Bulletin 2026/23**

(21) Numéro de dépôt: **21755533.3**

(22) Date de dépôt: **19.07.2021**

(51) Classification Internationale des Brevets (IPC):
***C09J 201/10*** (2006.01) ***C08L 29/10*** (2006.01)
***C08G 18/83*** (2006.01) ***C09J 7/38*** (2018.01)
***C09J 175/06*** (2006.01) ***C09J 175/08*** (2006.01)
***C08G 18/28*** (2006.01) ***C08G 65/336*** (2006.01)
***A61L 15/58*** (2006.01) ***C09J 171/02*** (2006.01)
***C08L 101/10*** (2006.01) ***C09J 143/04*** (2006.01)

(52) Classification Coopérative des Brevets (CPC):
(C-Sets disponibles)
**C08G 18/4825; A61L 15/585; C08G 18/10; C08G 18/289; C08G 18/837; C08L 101/10; C09J 7/38; C09J 175/06; C09J 175/08; C09J 201/10;** C09J 143/04; C09J 2483/00 (Cont.)

(86) Numéro de dépôt international:
**PCT/FR2021/051350**

(87) Numéro de publication internationale:
**WO 2022/018375 (27.01.2022 Gazette 2022/04)**

# (54) COMPOSITION ADHESIVE POUR LA FABRICATION DES ARTICLES IMPER-RESPIRANTS

KLEBSTOFFZUSAMMENSETZUNG ZUR HERSTELLUNG WASSERDICHTER ATMUNGSFÄHIGER GEGENSTÄNDE

ADHESIVE COMPOSITION FOR MAKING WATERPROOF BREATHABLE ARTICLES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.07.2020 FR 2007707**

(43) Date de publication de la demande:
**31.05.2023 Bulletin 2023/22**

(73) Titulaire: **Bostik SA**
**92800 Puteaux (FR)**

(72) Inventeur: **GARNIER, Claire**
**60280 VENETTE (FR)**

(74) Mandataire: **Arkema Patent**
**Arkema France**
**DRD-DPI**
**51, Esplanade du Général de Gaulle**
**CS 10478**
**92907 Paris La Défense Cedex (FR)**

(56) Documents cités:
**WO-A1-2013/136108**
**WO-A1-2014/120593**
**JP-A- H0 782 492**
**JP-A- H10 147 723**
**JP-A- S6 389 559**
**JP-B2- 3 473 049**
**KR-A- 20020 056 447**
**KR-A- 20100 055 161**
**US-A1- 2012 107 626**

Il est rappelé que: Dans un délai de neuf mois à compter de la publication de la mention de la délivrance du brevet européen au Bulletin européen des brevets, toute personne peut faire opposition à ce brevet auprès de l’Office européen des brevets, conformément au règlement d’exécution. L’opposition n’est réputée formée qu’après le paiement de la taxe d’opposition. (Art. 99(1) Convention sur le brevet européen).

(52) Classification Coopérative des Brevets (CPC):
(Cont.)

C-Sets
**A61L 15/585, C08L 29/10;**
**A61L 15/585, C08L 83/04;**
**C08G 18/10, C08G 18/289;**
**C08L 101/10, C08L 29/10;**
**C09J 201/10, C08L 29/10**

## Description

### Domaine de l'invention

La présente invention a pour objet une composition adhésive, et l'utilisation de ladite composition adhésive pour la préparation d'articles autoadhésifs.

### Arrière-Plan technique

Une composition adhésive thermofusible sensible à la pression (également dénommée composition de colle autoadhésive ou encore, en anglais, "Hot-Melt Pressure Sensitive Adhesives" ou HMPSA) est une composition conférant au support qui en est revêtu un pouvoir collant immédiat à température ambiante (souvent désigné sous le terme de "tack"). Ce tack permet avantageusement l'adhésion instantanée de cette composition à un substrat sous l'effet d'une pression légère et brève. Les HMPSA sont largement utilisées pour la fabrication d'articles autoadhésifs, tels que par exemple des étiquettes autoadhésives fixées sur des articles à des fins de présentation d'informations (code barre, dénomination, prix, etc.) et/ou à des fins décoratives, que ce soit lors de collages définitifs ou temporaires. Les HMPSA sont également mis en œuvre pour la fabrication de rubans autoadhésifs d'utilisations variées. Outre le ruban adhésif transparent largement utilisé dans la vie quotidienne, on peut citer par exemple : la mise en forme et l'assemblage d'emballages en carton ; la protection de surfaces pour les travaux de peinture, dans la construction ; la fixation et le maintien d'éléments divers tels que panneaux, briques, objets protubérants, dans la construction de bâtiments ou d'édifices ; la fixation et le maintien de parties métalliques ou plastiques ou en verre, plates ou ayant des profils spécifiques, tels des câbles électriques, des films plastiques, des vitres, des tôles, des inscriptions, des logos, des parties de sièges, des tableaux de bord, des parois plastiques ou textiles, des conduits ou des tuyaux de circulation de fluides, notamment dans l'industrie des transports ; le collage des moquettes par rubans adhésifs à double face dans le domaine du bâtiment.

Les articles autoadhésifs imper-respirants peuvent être utilisés dans des applications médicales telles que des bandages, des pansements, des électrodes, des plâtres, dans des applications vestimentaires telles que des vêtements respirants pour faire face aux intempéries ou des vêtements secs respirants exposés à un environnement humide et dans des applications de construction tels que les toitures ou les revêtements muraux, ou les systèmes étanches de châssis de fenêtre qui doivent encore être respirants pour que l'humidité soit rejetée dans l'environnement.

En particulier, les matériaux adhésifs utilisés par exemple dans le domaine médical, tels que les rubans médicaux, les pansements de soins des plaies et les bandages grand public, nécessitent un taux de transmission de vapeur d'humidité (en anglais « Moisture Vapour Transmission rate » ou MVTR) élevé pour permettre l'évacuation de l'humidité générée par la peau ou par l'exsudat de la plaie. Un MVTR élevé du produit adhésif empêche l'humidité d'être piégée sous le pansement, ce qui pourrait autrement provoquer une macération de la peau.

Ainsi, les articles respirants doivent remplir certaines conditions telles qu'un aspect homogène, une résistance au vent, une haute perméabilité à la vapeur d'eau, une certaine élasticité, ainsi qu'une capacité d'adhérer à des différents substrats.

Des compositions adhésives adaptées à la préparation d'articles autoadhésifs sont déjà connues.

Par exemple, le document WO 2013/136108 A1 concerne notamment une composition adhésive comprenant au moins un polymère silylé, au moins une résine tackifiante et au moins un catalyseur, et son utilisation pour fabriquer des articles imper-respirants.

Le document WO 2019/115952 A1 concerne notamment une composition adhésive multicomposante comprenant une composition A comprenant au moins un polymère silylé, ayant au moins un groupe alkoxysilane hydrolysable, et au moins une résine tackifiante ; et une composition B comprenant au moins un catalyseur et au moins un composé C. Le composé C est choisi parmi un composé C1 ayant une masse moléculaire moyenne en nombre allant de 300 g/mol à 500 000 g/mol et un composé C2 ayant une tension de vapeur à 20°C supérieure ou égale à 0,08 kPa.

Le document WO 2019/186014 A1 concerne notamment une composition adhésive comprenant au moins un polymère silylé réticulable et une composition catalytique comprenant une amine tertiaire et un composé organométallique.

Le document WO 03/087254 A2 concerne notamment une composition adhésive hydrophile caractérisée en ce qu'elle comprend un élastomère thermoplastique du type copolymère séquence poly(styrène-oléfine-styrène), un produit tackifiant, un plastifiant liquide, de l'eau et un copolymère amphiphile.

Le document WO 2020/016581 A1 concerne notamment une composition comprenant des polymères comprenant des groupements silylés fonctionnalisés par des groupes d'urée et d'amine et une résine tackifiante, ces compositions étant utilisées comme des adhésives thermofusibles sensibles à la pression.

Des exemples de compositions contenant au moins un polymère silylé, au moins un catalyseur de réticulation et au moins un copolymère de polyvinyléther sont décrits dans JP S63 89559 A, JP H07 82492 A, JP 3 473049 B2, et JP H10 147723 A.

Ces divers articles autoadhésifs imper-respirants, et les compositions adhésives qui les composent, présentent des propriétés adhésives satisfaisantes. Cependant, ces articles, et les compositions qui les composent, ne sont pas nécessairement adaptés aux applications médicales. En particulier, ces articles, et les compositions qui les composent, tels que les rubans médicaux, les bandages et les pansements, ne sont pas nécessairement adaptés à une application prolongée sur la peau ou une plaie. En effet, la pose prolongée de ces articles peut entraîner une macération de la peau ou de la plaie, ce qui peut générer un inconfort pour le patient, ralentir la cicatrisation, etc.

Il existe donc un réel besoin de fournir une composition adhésive, qui permet la fabrication des articles autoadhésifs adaptés à un usage médical et présentant une perméabilité à la vapeur d'eau améliorée, sans compromettre les bonnes propriétés d'adhésion. Il existe également un réel besoin de fournir une composition adhésive, qui permet la fabrication d'articles autoadhésifs - tels que les rubans médicaux, les bandages ou les pansements - adaptés à une application prolongée sur la peau ou la plaie. Il existe également un réel besoin de fournir une composition adhésive, qui permet la fabrication des articles autoadhésifs - tels que les rubans médicaux, les bandages ou les pansements - limitant voire empêchant la macération de la peau ou de la plaie sur laquelle ils sont appliqués. Il existe également un réel besoin de fournir une composition adhésive, qui permet la fabrication des articles autoadhésifs - tels que les rubans médicaux, les bandages ou les pansements - facilitant la cicatrisation des plaies. Il existe également un réel besoin de fournir une composition adhésive, qui permet la fabrication des articles autoadhésifs adaptés au marché de la construction et présentant une perméabilité à la vapeur d'eau améliorée, sans compromettre les bonnes propriétés d'adhésion.

## Résumé de l'invention

L'invention concerne en premier lieu une composition adhésive comprenant :au moins un polymère silylé comprenant au moins un groupement alkoxisilane hydrolysable ;

au moins un composé polyvinyléther ; et
au moins un catalyseur de réticulaltion, dans laquelle le composé polyvinyléther est un homopolymère choisi parmi le poly(méthylvinyléther), le poly(éthylvinyléther), le poly(butylvinyléther), le poly(isobutylvinyléther), le poly(isopropylvinyléther), le poly(propylvinyléther), le poly(octylvinyléther), et leurs mélanges.

Dans des modes de réalisation, le polymère silylé est choisi parmi :

un polymère silylé de formule générale [Chem 2]

$$(R^5O)_{3-p}(R^4)_pSi-R^6-O-R^2-O-R^6-Si(R^4)p(OR^5)_{3-p}$$

un polymère silylé de formule générale [Chem 3]

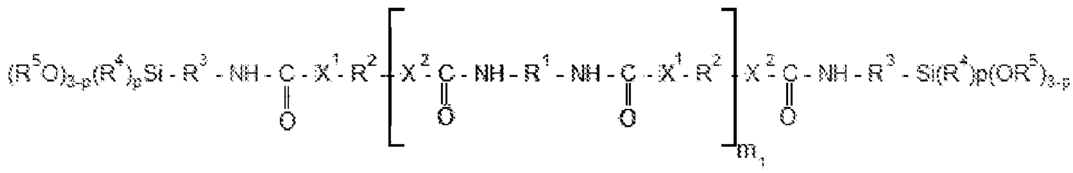

un polymère silylé de formule générale [Chem 4]

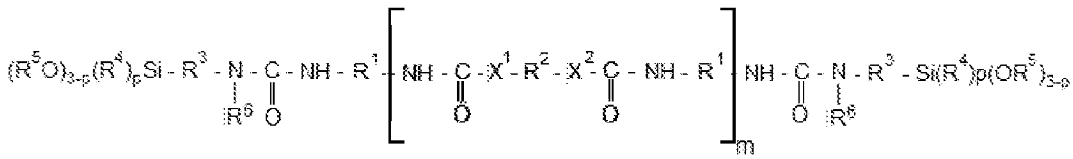

un polymère silylé de formule générale [Chem 5]

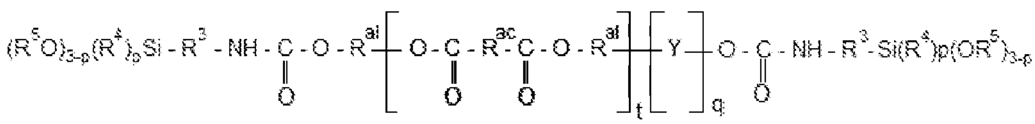

dans lesquelles :

$X^1$ et $X^2$ représentent, indépendamment les uns des autres, un atome d'oxygène ou un groupement -NH- ;
$R^1$ représente un radical divalent hydrocarboné comprenant de 5 à 15 atomes de carbone qui peut être aromatique ou aliphatique, linéaire, ramifié ou cyclique ;
$R^0$ représente un radical divalent alkylène linéaire ou ramifié comprenant de 3 à 6 atomes de carbone ;
$R^3$ représente un radical divalent alkylène linéaire ou ramifié comprenant de 1 à 6 atomes de carbone, de préférence $R^3$ représentant méthylène ou n-propylène ;
$R^2$ représente un bloc polyéther $-R^{pe}-[OR^{pe}]_n-$ dans lequel $R^{pe}$ représente un radical divalent alkylène linéaire ou ramifié comprenant de 2 à 4 atomes de carbone ;
$R^4$ et $R^5$, identiques ou différents, représentent chacun un radical alkyl linéaire ou ramifié comprenant de 1 à 4 atomes de carbone ;
$R^6$ représente un atome d'hydrogène, un radical phényle, un radical alkyle linéaire, ramifié ou cyclique comprenant de 1 à 6 atomes de carbone, ou un radical 2-succinate de formule générale [Chem 6]

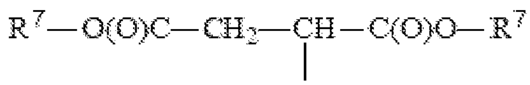

dans laquelle $R^7$ est un radical alkyle linéaire ou ramifié comprenant de 1 à 6 atomes de carbone ;
n est un nombre entier tel que la masse moléculaire moyenne en nombre du bloc polyéther $-[OR^{pe}]_n-$ va de 300 g/mol à 40 000 g/mol dans les polymères de formules générales [Chem 2], [Chem 3] et [Chem 4] ;
$m_1$ est zéro ou un nombre entier ;
$m_1$ sont tel que la masse moléculaire moyenne en nombre du polymère de formule générale [Chem 3] va de 500 g/mol à 50 000 g/mol, de préférence de 700 g/mol à 20 000 g/mol ;
m est un nombre entier différent de zéro ;
m est tel que la masse moléculaire moyenne en nombre du polymère de formule générale [Chem 4] va de 500 g/mol à 50 000 g/mol, de préférence de 700 g/mol à 20 000 g/mol ;
p est un nombre entier égal à 0, 1 ou 2, p étant de préférence 0 ou 1 ;
$R^{al}$ représente un radical hydrocarboné divalent issu d'un diol par remplacement de chacun des deux groupes hydroxyles par une valence libre ou représente le radical $R^2$;
$R^{ac}$ représente un radical hydrocarboné divalent issu d'un acide dicarboxylique par remplacement de chacun des deux groupes carboxyles COOH par une valence libre ;
$[Y]_q$ représente un motif de répétition de formule générale [Chem 28]

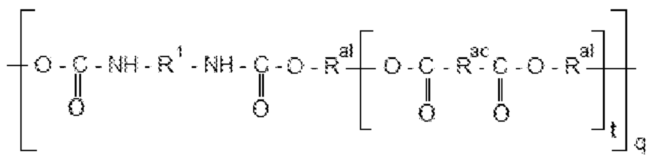

t est un nombre tel que le polyester diol de formule générale [Chem 7]

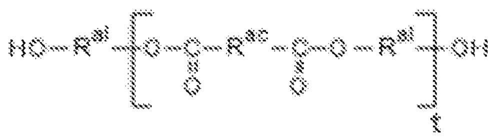

a un indice d'hydroxyle IOH compris entre 4 et 60 mg KOH/g ; q est un nombre entier différent de zéro ;
t et q sont tels que la masse moléculaire moyenne en nombre du polymère de formule générale [Chem 5] est comprise entre 400 g/mol et 50 000 g/mol, ledit indice hydroxyle IOH et ladite masse moléculaire moyenne étant déterminés selon la norme ISO 14900:200

Dans des modes de réalisation, le catalyseur de réticulation est choisi dans le groupe constitué des amines, des composés organo-métalliques, des acides et de leurs dérivés, et de leurs mélanges.
Dans des modes de réalisation, la composition comprend en outre une résine tackifiante ; de préférence une résine tackifiante choisie parmi les résines terpènes-phénoliques, les résines d'hydrocarbures, les résines de colophane ; les résines acryliques et leurs mélanges.

Dans des modes de réalisation, le composé polyvinyléther présente une valeur K de 40 à 120, et de préférence de 40 à 70, ladite valeur K étant mesurée selon la norme ISO 1628-1.

Dans des modes de réalisation, le composé polyvinyléther présente une température de transition vitreuse de -60 à 0°C, et de préférence de -50 à - 5°C. -5°C, ladite température de transition vitreuse étant mesurée selon la norme NF EN ISO 11357-2.

Dans des modes de réalisation, le composé polyvinyléther a une teneur de 1 à 60 % en poids, de préférence de 5 à 40 % en poids et de préférence de 5 à 25 % en poids par rapport au poids total de la composition.

Dans des modes de réalisation, la composition comprend en outre au moins un silsesquioxane.

Dans des modes de réalisation, ladite composition est une composition mono-composante.

Dans des modes de réalisation, ladite composition est une composition bi-composante comprenant :

une partie A comprenant ledit au moins un polymère silylé ; et
une partie B comprenant ledit au moins un catalyseur de réticulation ledit au moins un composé polyvinyléther étant présent dans la partie A et/ou dans la partie B de la composition.

L'invention concerne également l'utilisation de la composition décrite ci-dessus comme adhésif.

L'invention concerne également un article auto-adhésif comprenant au moins une couche support et au moins une couche de la composition décrite ci-dessus.

Dans des modes de réalisation, ledit article est choisi parmi les pansements, les bandages et les rubans médicaux.

La présente invention permet de répondre aux besoins exprimés ci-dessus. Elle fournit plus particulièrement une composition permettant de fournir une composition qui permet la fabrication des articles présentant une bonne perméabilité à la vapeur d'eau améliorée, sans compromettre les bonnes propriétés d'adhésion.

Cela est accompli grâce à la composition selon l'invention. Plus particulièrement, les inventeurs ont démontré de manière surprenante que la composition adhésive selon l'invention - en particulier une composition comprenant au moins polymère silylé, au moins un composé polyvinyléther et au moins un catalyseur de réticulation - permet d'obtenir des articles autoadhésifs présentant une perméabilité à la vapeur améliorée, sans compromettre pour autant de bonnes propriétés d'adhésion sur un substrat. En effet, la présence du composé polyvinyléther permet d'obtenir une bonne compatibilité avec les autres constituants de la composition et plus particulièrement avec les chaînes du polymère silylé (présentant déjà une bonne perméabilité à la vapeur d'eau), ce qui a comme résultat une amélioration de la perméabilité à la vapeur d'eau de la composition adhésive. Par « bonne compatibilité » on entend ici une stabilité au stockage et dans le temps de la composition.

## Description détaillée

L'invention est maintenant décrite plus en détail et de façon non limitative dans la description qui suit.

### Composition adhésive

Dans un premier aspect, la présente invention concerne une composition adhésive comprenant au moins un polymère silylé comprenant au moins un groupement alkoxysilane hydrolysable, au moins un composé polyvinyléther, et au moins un catalyseur de réticulation. Des différents additifs peuvent également être présents dans la composition adhésive.

### Polymère silylé

Le polymère silylé comprend au moins un groupe alkoxysilane hydrolysable, de préférence terminal, et de préférence au moins deux groupes alkoxysilane hydrolysables, préférentiellement terminaux.

Le polymère silylé peut être un polymère comprenant au moins un, de préférence au moins deux groupes, en particulier terminal (aux), de formule générale [Chem 1]

$$-Si(R^4)_p(OR^5)_{3-p}$$

dans laquelle :

$R^4$ et $R^5$, identiques ou différents, représentent chacun un radical alkyl linéaire ou ramifié comprenant de 1 à 4 atomes de carbone ; et
p est un nombre entier égal à 0, 1 ou 2.

Lorsque p = 2, les groupes $R^4$ peuvent être identiques ou différents. Lorsque p = 1 ou p = 0, les groupes $R^5$ peuvent être identiques ou différents. Le polymère silylé comprenant au moins un groupe alkoxysilane hydrolysable, de préférence au moins deux, peut posséder une chaîne principale choisie parmi les chaînes principales suivantes : polyéther, polyester, polyester-polyéther-polyester, polyéther-polyester-polyéther, polyoléfine, polycaprolactone, polyacrylate, polycarbonate, poly(éther-carbonate), poly(ester-carbonate), polyacétal, polyesteramide, polythioéther, polyuréthane, polyester-polyuréthane, polyéther-polyuréthane, polyéther-polyester-polyuréthane, polyoléfine-polyuréthane, polyéther-polyoléfine-polyuréthane, polyurée ou poly(uréthane-urée).

De préférence, les polymères silylés comportant au moins un groupe alkoxysilane hydrolysable sont choisis parmi les polyéther-polyuréthane silylés, les polyéthers silylés, les polyester silylés, les polyester-polyuréthane silylés, les polyéther-polyester-polyuréthane silylés, les polyurées silylées, les poly(uréthane-urée) silylés et leurs mélanges.

Le polymère silylé peut avoir une masse moléculaire moyenne en nombre (Mn) allant de 500 à 50 000 g/mol, de préférence allant de 700 à 30 000 g/mol, avantageusement de 1 000 à 25 000 g/mol, en particulier de 1 000 à 21 000 g/mol.

La masse moléculaire moyenne en nombre des polymères silylés peut être mesurée par des méthodes bien connues de l'homme du métier, par exemple par chromatographie d'exclusion stérique en utilisant des étalons de type polystyrène.

Le polymère silylé comportant au moins un groupe alkoxysilane hydrolysable peut être choisi parmi les polymères de formules générales [Chem 2], [Chem 3], [Chem 4] ou [Chem 5] telles que définies ci-dessous, et leurs mélanges :

[Chem 2] $(R^5O)_{3-p}(R^4)_pSi\text{-}R^0\text{-}O\text{-}R^2\text{-}O\text{-}R^0\text{-}Si(R^4)p(OR^5)_{3-p}$

[Chem 3]

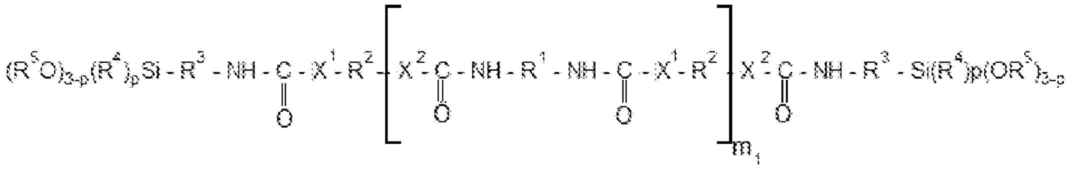

[Chem 4]

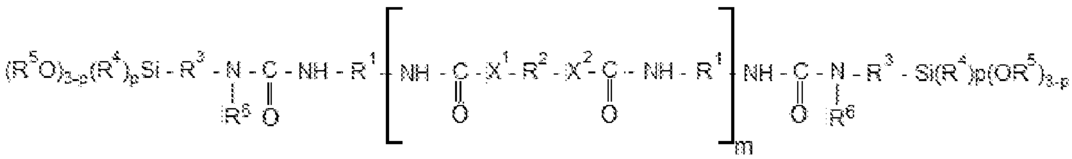

[Chem 5]

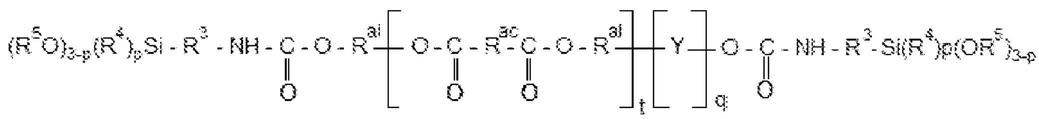

dans lesquelles :

$X^1$ et $X^2$ représentent, indépendamment les uns des autres, un atome d'oxygène ou un groupement -NH- ;
$R^1$ représente un radical divalent hydrocarboné comprenant de 5 à 15 atomes de carbone qui peut être aromatique ou aliphatique, linéaire, ramifié ou cyclique ;
R0 représente un radical divalent alkylène linéaire ou ramifié comprenant de 3 à 6 atomes de carbone ;
$R^3$ représente un radical divalent alkylène linéaire ou ramifié comprenant de 1 à 6 atomes de carbone, de préférence $R^3$ représentant méthylène ou n-propylène ;
$R^2$ représente un bloc polyéther $-R^{pe}-[OR^{pe}]_n-$ dans lequel $R^{pe}$ représente un radical divalent alkylène linéaire ou ramifié comprenant de 2 à 4 atomes decarbone ;

n est un nombre entier tel que la masse moléculaire moyenne en nombre du bloc polyéther $-[OR^{pe}]_n-$ va de 300 g/mol à 40 000 g/mol dans les polymères de formules générales [Chem 2], [Chem 3] et [Chem 4] ;

$R^4$ et $R^5$, étant tels que définis ci-dessus ;

$R^6$ représente un atome d'hydrogène, un radical phényle, un radical alkyle linéaire, ramifié ou cyclique comprenant de 1 à 6 atomes de carbone, ou un radical 2-succinate de formule générale [Chem 6]

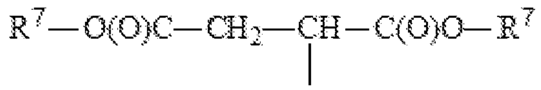

dans laquelle $R^7$ est un radical alkyle linéaire ou ramifié comprenant de 1 à 6 atomes de carbone ;

$m_1$ est zéro ou un nombre entier ;

$m_1$ est tel que la masse moléculaire moyenne en nombre du polymère de formule générale [Chem 3] va de 500 g/mol à 50 000 g/mol, de préférence de 700 g/mol à 20 000 g/mol ;

m est un nombre entier différent de zéro ;

m est tel que la masse moléculaire moyenne en nombre du polymère de formule générale [Chem 4] va de 500 g/mol à 50 000 g/mol, de préférence de 700 g/mol à 20 000 g/mol ;

p est tel que défini ci-dessus, p étant de préférence 0 ou 1 ;

$R^{al}$ représente un radical hydrocarboné divalent issu d'un diol par remplacement de chacun des deux groupes hydroxyles par une valence libre ou représente le radical $R^2$;

$R^{ac}$ représente un radical hydrocarboné divalent issu d'un acide dicarboxylique par remplacement de chacun des deux groupes carboxyles -COOH par une valence libre ;

$[Y]_q$ représente un motif de répétition de formule générale [Chem 28]

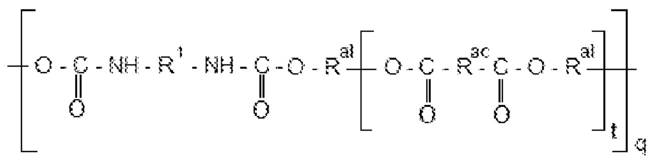

t est un nombre tel que le polyester diol de formule [Chem 7]

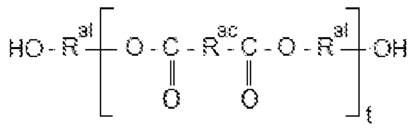

a un indice d'hydroxyle $I_{OH}$ compris entre 4 et 60 mg KOH/g ;

q est un nombre entier différent de zéro ; et

t et q sont tels que la masse moléculaire moyenne en nombre du polymère de formule générale [Chem 5] est comprise entre 400 g/mol et 50 000 g/mol. Selon certains modes de réalisation, $X^1$ et $X^2$ sont tous les deux un atome d'oxygène.

Alternativement, $X^1$ et $X^2$ sont tous les deux un groupement -NH-.

Encore alternativement, l'un des $X^1$ et $X^2$ peut être un atome d'oxygène et l'autre de $X^1$ et $X^2$ peut être un groupement -NH-.

De préférence, le radical $R^1$ des formules générales [Chem 3], [Chem 4] et [Chem 5] est choisi parmi l'un des radicaux divalents suivants dont les formules ci-dessous font apparaître les 2 valences libres :

le radical divalent dérivé de l'isophorone diisocyanate (IPDI) [Chem 8]

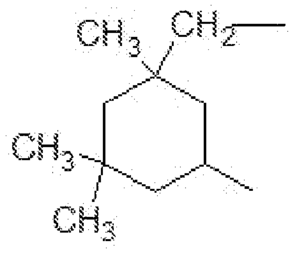

le radical divalent dérivé des 4,4'- et 2,4'-dicyclohexylméthane diisocyanate (HMDI) de formule [Chem 9]

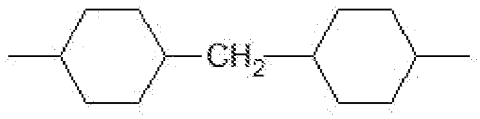

ou de formule [Chem 10]

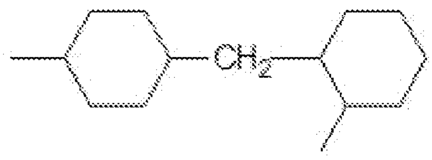

le radical dérivé des 2,4- et 2,6-toluène diisocyanate (TDI) de formule [Chem 11]

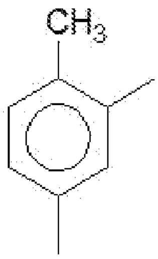

ou de formule [Chem 12]

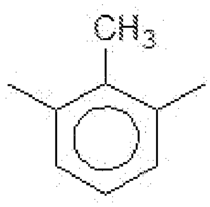

le radical dérivé des 4,4'- et 2,4'-diphénylméthane diisocyanate (MDI) de formule [Chem 13]

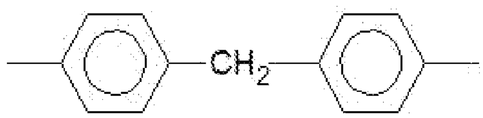

ou de formule [Chem 14]

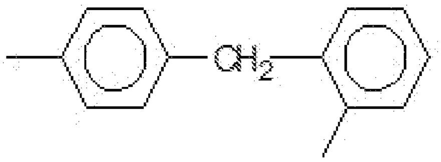

le radical dérivé du m-xylylène diisocyanate (m-XDI) de formule [Chem 15]

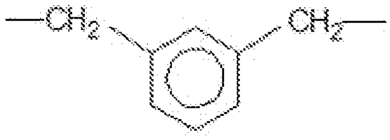

le radical dérivé de l'hexamethylène diisocyanate (HDI) de formule

[Chem 16] $-(CH_2)_6-$

De préférence, le radical $R^1$ des formules générales [Chem 3], [Chem 4] et [Chem 5] est le radical divalent dérivé de l'isophorone diisocyanate ou du xylylène diisocyanate.

Les polymères de formule générale [Chem 3] peuvent par exemple être obtenus selon un procédé décrit dans les documents EP 2336208 A1 et WO 2009/106699 A2.

Parmi les polymères répondant à la formule générale [Chem 3], on peut par exemple citer :

- GENIOSIL® STP-E10 (disponible auprès de WACKER) : polyéther comprenant deux groupes [Chem 1] de type diméthoxy ($m_1$ égal à 0, p égal à 1 et $R^4$ et $R^5$ représentent un groupement méthyle) présentant une masse moléculaire moyenne en nombre d'environ 8 889 g/mol où R3 représente un groupement méthylène ;
- GENIOSIL® STP-E30 (disponible auprès de WACKER) : polyéther comprenant deux groupes de formule générale [Chem 1] de type diméthoxy ($m_1$ égal à 0, p égal à 1 et $R^4$ et $R^5$ représentent un groupement méthyle) présentant une masse moléculaire moyenne en nombre d'environ 14 493 g/mol où $R^3$ représente un groupement méthylène ;
- SPUR+® 1050MM (disponible auprès de MOMENTIVE) : polyéther-polyuréthane comprenant deux groupes de formule générale [Chem 1] de type triméthoxy ($m_1$ différent de 0, p égal à 0 et $R^5$ représente un groupement méthyle) présentant une masse moléculaire moyenne en nombre d'environ 16 393 g/mol où $R^3$ représente un groupement n-propylène ;
- SPUR+® Y-19116 (disponible auprès de MOMENTIVE) : polyéther-polyuréthane comprenant deux groupes de formule générale [Chem 1] de type triméthoxy ($m_1$ différent de 0 et $R^5$ représente un groupement méthyle) présentant une masse moléculaire moyenne en nombre allant de 15 000 à 17 000 g/mol où $R^3$ représente un groupement n-propylène ;
- DESMOSEAL® S XP 2636 (disponible auprès de BAYER) : polyéther comprenant deux groupes de formule générale [Chem 1] de type triméthoxy ($m_1$ égal à 0, p égal à 0 et $R^5$ représente un groupement méthyle) présentant une masse moléculaire moyenne en nombre d'environ 15 038 g/mol où $R^3$ représente un groupement n-propylène.

Les polymères de formule générale [Chem 2] peuvent être obtenus par hydrosilylation de polyéther diallyléther selon un procédé décrit par exemple dans le document EP 1829928 A1.

Parmi les polymères répondant à la formule générale [Chem 2], on peut citer :

- le MS POLYMER™ S303H (disponible auprès de KANEKA) correspondant à un polyéther comprenant deux groupes de formule générale [Chem 1] de type diméthoxy (p est égal à 1 et $R^4$ représente un groupement méthyle) ayant une masse moléculaire moyenne en nombre d'environ 22 000 g/mol et une viscosité de 12,5 Pa.s à 23°C ; et
- le MS POLYMER™ S227 (disponible auprès de KANEKA) correspondant à un polyéther comprenant deux groupes de formule générale [Chem 1] de type diméthoxy (p est égal à 1, $R^5$ et $R^4$ représentent chacun un groupement méthyle) ayant une masse moléculaire moyenne en nombre d'environ 27 000 g/mol, et une viscosité de 34 Pa.s à 23°C.

Les polymères de formule générale [Chem 4] peuvent être obtenus selon le procédé suivant (dans le cas où le polymère de formule générale [Chem 4] est un polyuréthane silylé) :

- réaction d'un polyéther polyol de formule générale [Chem 17]

$HO-R^2-OH$

avec un excès stœchiométrique de diisocyanate de formule $NCO-R^1-NCO$ pour former un bloc polyuréthane-polyéther ayant au moins deux groupes terminaux -NCO, ledit bloc comprenant de préférence de 1,5 % à 1,9 % en poids de groupement -NCO, et puis

- réaction entre un bloc obtenu à l'étape précédente avec une quantité stœchiométrique ou un léger excès d'une $\alpha$-, $\beta$- ou $\gamma$-aminosilane ayant la formule générale [Chem 18]

$(R^5O)_{3-p}(R^4)_pSi-R^3-NHR^6$

Un tel procédé est par exemple décrit dans WO 2013/136108 A1.

Parmi les polymères répondant à la formule générale [Chem 4], on peut par exemple citer :

- le SPUR+ 1015 LM (disponible auprès de MOMENTIVE) correspondant à un polyéther-polyuréthane comprenant deux groupes de [Chem 1] de type triméthoxy (p est égal à 0, $R^5$ représente un groupement méthyle) ayant une masse moléculaire en nombre d'environ 25 000 g/mol et une viscosité de 50 Pa à 23°C.

Les polymères de formule générale [Chem 4] peuvent être obtenus selon le procédé suivant (dans le cas où le polymère de formule générale [Chem 4] est une polyurée silylée) :

- réaction d'une diamine de formule générale [Chem 19]

$H_2N-R^2-NH_2$

avec un excès stœchiométrique de diisocyanate de formule $NCO-R^1-NCO$ pour former un bloc polyurée-polyéther ayant au moins deux groupes terminaux -NCO, ledit bloc comprenant de préférence de 1,5 % à 1,9 % en poids de groupement -NCO, et puis

- réaction entre un bloc obtenu à l'étape précédente avec une quantité stœchiométrique ou un léger défaut d'une $\alpha$-, $\beta$- ou $\gamma$-aminosilane ayant la formule générale [Chem 18]

$(R^5O)_{3-p}(R^4)_pSi-R^3-NHR^6$

Un tel procédé est par exemple décrit dans WO 2020/016581 A1.

Les polymères de formule générale [Chem 5] peuvent être ceux décrits dans la demande EP 2865694 A1. Ils peuvent être préparés selon le procédé décrit dans cette demande.

Le polymère de formule générale [Chem 5] peut être un premier polymère de formule générale [Chem 5] spécifique dans laquelle :

$R^{al}$ représente un radical hydrocarboné divalent qui est dérivé d'un alcool gras dimérisé par remplacement de chacun des 2 groupes hydroxyle par une valence libre, ledit alcool ayant un indice d'hydroxyle $I_{OH}$ compris entre 200 et 220 mg KOH/g ;

$R^{ac}$ représente un radical hydrocarboné divalent qui est dérivé d'un acide gras dimérisé par remplacement de chacun des 2 groupes carboxyles -COOH par une valence libre, ledit acide ayant un indice d'acide $I_A$ compris entre 190 et 200 mg KOH/g :

t est préférentiellement un nombre tel que le polyester diol de formule générale [Chem 7]

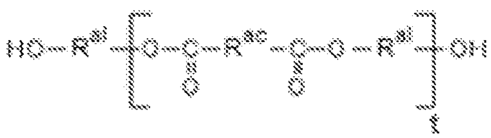

a un indice d'hydroxyle $I_{OH}$ compris entre 45 et 55 mg KOH/g.

Ce premier polymère de formule générale [Chem 5] spécifique peut être obtenu selon le procédé décrit dans la demande EP 2865 728 A1 (en particulier aux pages 5 à 9).

De préférence, ce premier polymère de formule générale [Chem 5] spécifique a une masse moléculaire moyenne en nombre allant de 900 à 15 000 g/mol.

Le polymère de formule générale [Chem 5] peut être un second polymère de formule générale [Chem 5] spécifique dans laquelle :

$R^{al}$ représente un radical hydrocarboné divalent issu d'un diol saturé, par remplacement de chacun des deux groupes hydroxyles par une valence libre, ledit diol ayant un indice d'hydroxyle $I_{OH}$ supérieur à 220 mg KOH/g ;
$R^{ac}$ représente un radical hydrocarboné divalent issu d'un acide dicarboxylique saturé, par remplacement de chacun des deux groupes carboxyles -COOH par une valence libre, ledit acide ayant un indice d'acide $I_A$ supérieur à 200 mg KOH/g
t est préférentiellement un nombre tel que le polyester diol de formule générale [Chem 7]

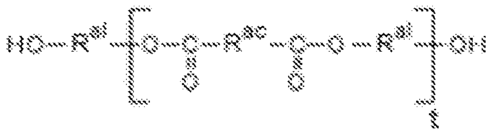

a un indice d'hydroxyle $I_{OH}$ compris entre 4 et 24 mg KOH/g, en particulier entre 9 et 24 mg KOH/g.

Le second polymère de formule générale [Chem 5] spécifique peut être obtenu par un procédé qui comprend plusieurs étapes séquentielles :

La première étape du procédé d'obtention du second polymère de formule générale [Chem 5] spécifique consiste en la réparation d'un polyester de formule générale [Chem 7] ayant un indice hydroxyle $I_{OH}$ compris entre 4 et 24 mg KOH/g
Le polyester de formule générale [Chem 7] peut être préparé par une réaction de polycondensation entre :

au moins un acide dicarboxylique saturé ayant un indice d'acide $I_A$ supérieur à 200 mg KOH/g ; et
au moins un diol saturé ayant un indice d'hydroxyle $I_{OH}$ supérieur à 220 mg KOH/g.

Dans le présent texte :
l'indice d'acide $I_A$ d'un acide dicarboxylique est le nombre de fonction carboxylique par gramme d'acide, ledit nombre étant exprimé sous la forme d'équivalent en milligrammes de KOH nécessaire pour neutraliser l'acidité de 1 gramme de d'acide, déterminé par titrimétrie, ledit nombre étant relié à la masse moléculaire moyenne en nombre M dudit acide par la relation [Math 1] suivante

$$I_A = (56,1 \times 2 \times 1000) / M$$

l'indice hydroxyle $I_{OH}$ d'un diol est le nombre de fonctions hydroxyle par gramme de diol, ledit nombre étant exprimé sous la forme d'équivalent en milligrammes de KOH utilisé dans le dosage des fonctions hydroxyles, déterminé par titrimétrie selon la norme ISO 14900:2001, ledit nombre étant relié à la masse moléculaire moyenne en nombre M' dudit diol par la relation

[Math 2]

$$I_{OH} = (56,1 \times 2 \times 1000) / M'$$

De préférence, l'(les) acide(s) dicarboxylique(s) saturé(s) a (ont) un indice d'acide $I_A$ supérieur ou égal à 300 mg KOH/g, de préférence supérieur ou égal à 400 mg KOH/g, préférentiellement supérieur ou égal à 500 mg KOH/g, en particulier supérieur ou égal à 700 mg KOH/g, et avantageusement supérieur ou égal à 800 mg KOH/g. De préférence, l'(les) acide(s) dicarboxylique(s) saturé(s) a (ont) un indice d'acide IA égal à 555 mg KOH/g ou égal à 768 mg KOH/g.

L'acide dicarboxylique peut être linéaire ou ramifié, de préférence linéaire, aliphatique ou cycloaliphatique.

L'acide dicarboxylique selon l'invention peut être choisi dans le groupe constitué de l'acide malonique, de l'acide succinique, de l'acide fumarique, de l'acide glutarique, de l'acide adipique, de l'acide 1,3- ou 1,4-cyclohexane dicarboxylique, de l'acide 3-méthyl-1,5-pentanedicarboxylique, de l'acide 1,10-décanedicarboxylique, de l'acide 1,12-dodécanedicarboxylique, de l'acide 1,18-octadécanedicarboxylique, de l'acide méthyltétrahydrophthalique, de l'acide hexahydrophthalique, de l'acide tétrahydrophthalique, de l'acide azélaique, de l'acide sébacique et de leurs mélanges.

De préférence, l'acide dicarboxylique est l'acide adipique ou l'acide sébacique.

De préférence, le(s) diol(s) saturé(s) a(ont) un indice d'hydroxyle $I_{OH}$ supérieur ou égal à 500 mg KOH/g, de préférence supérieur ou égal à 700 mg KOH/g, encore plus préférentiellement supérieur ou égal à 900 mg KOH/g.

Le diol mis en œuvre peut être aromatique ou aliphatique (de préférence aliphatique), linéaire ou ramifié, de préférence ramifié.

Le diol selon l'invention peut être choisi dans le groupe constitué de l'éthylène glycol (CAS : 107-21-1), du diéthylène glycol, du triéthylène glycol, du tétraéthylène glycol, du 1,2-propanediol, du dipropylène glycol, du tripropylène glycol, du tétrapropylène glycol, du 1,6-hexanediol, du 3-éthyl-2-méthyl-1,5-pentanediol, du 2-éthyl-3-propyl-1,5-pentanediol, du 2,4-diméthyl-3-éthyl-1,5-pentanediol, du 2-éthyl-4-méthyl-3-propyl-1,5-pentadiol, du 2,3-diéthyl-4-méthyl-1,5-pentanediol, du 3-éthyl-2,2,4-triméthyl-1,5-pentadiol, 2,2-diméthyl-4-éthyl-3-propyl-1,5-pentanediol, 2-méthyl-2-propyl-1,5-pentanediol, du 2,4-diméthyl-3-éthyl-2-propyl-1,5-pentanediol, du 2,3-dipropyl-4-éthyl-2-méthyl-1,5-pentanediol, du 2-butyl-2-éthyl-1,5-pentanediol, du 2-butyl-2,3-diéthyl-4-méthyl-1,5-pentanediol, du 2-butyl-2,4-diéthyl-3-propyl-1,5-pentanediol, du 3-butyl-2-propyl-1,5-pentanediol, du 2-méthyl-1,5-pentanediol (CAS : 42856-62-2), du 3-méthyl-1,5-pentanediol (MPD, CAS : 4457-71-0), du 2,2-diméthyl-1,3-pentanediol (CAS : 2157-31-5), du 2,2-diméthyl-1,5-pentanediol (CAS : 3121-82-2), du 3,3-diméthyl-1,5-pentanediol (CAS : 53120-74-4), du 2,3-diméthyl-1,5-pentanediol (CAS : 81554-20-3), du 2,2-diméthyl-1,3-propanediol (Néopentylglycol - NPG, CAS : 126-30-7), du 2,2-diethyl-1,3-propanediol (CAS : 115-76-4), du 2-méthyl-2-propyl-1,3-propanediol (CAS : 78-26-2), du 2-butyl-2-éthyl-1,3-propanediol (CAS : 115-84-4), du 2-méthyl-1,3-propanediol (CAS : 2163-42-0), du 2-benzyloxy-1,3-propanediol (CAS : 14690-00-7), du 2,2-dibenzyl-1,3-propanediol (CAS : 31952-16-6), du 2,2-dibutyl-1,3-propanediol (CAS : 24765-57-9), du 2,2-diisobutyl-1,3-propanediol, du 2,4-diéthyl-1,5-pentanediol, du 2-éthyl-1,6-hexanediol (CAS : 15208-19-2), du 2,5-diméthyl-1,6-hexanediol (CAS : 49623-11-2), du 5-méthyl-2-(1-methylethyl)-1,3-hexanediol (CAS : 80220-07-1), du 1,4-diméthyl-1,4-butanediol, du 1,5-hexanediol (CAS : 928-40-5), du 3-méthyl-1,6-hexanediol (CAS : 4089-71-8), du 3-tert-butyl-1,6-hexanediol (CAS : 82111-97-5), du 1,3-heptanediol (CAS : 23433-04-7), du 1,2-octanediol (CAS : 1117-86-8), du 1,3-octanediol (CAS : 23433-05-8), du 2,2,7,7-tétraméthyl-1,8-octanediol (CAS : 27143-31-3), du 2-méthyl-1,8-octanediol (CAS : 109359-36-6), du 2,6-diméthyl-1,8-octanediol (CAS : 75656-41-6), du 1,7-octanediol (CAS : 3207-95-2), du 4,4,5,5-tétraméthyl-3,6-dioxa-1,8-octanediol (CAS : 76779-60-7), du 2,2,8,8-tétraméthyl-1,9-Nonanediol (CAS : 85018-58-2), du 1,2-nonanediol (CAS : 42789-13-9), du 2,8-diméthyl-1,9-nonanediol (CAS : 40326-00-9), du 1,5-nonanediol (CAS : 13686-96-9), du 2,9-diméthyl-2,9-dipropyl-1,10-décanediol (CAS : 85018-64-0), du 2,9-dibutyl-2,9-diméthyl-1,10-decanediol (CAS : 85018-65-1), du 2,9-diméthyl-2,9-dipropyl-1,10-décanediol (CAS : 85018-64-0), du 2,9-diéthyl-2,9-diméthyl-1,10-décanediol (CAS : 85018-63-9), du 2,2,9,9-tétraméthyl-1,10-decanediol (CAS : 35449-36-6), du 2-nonyl-1,10-décanediol (CAS : 48074-20-0), du 1,9-décanediol (CAS : 128705-94-2), du 2,2,6,6,10,10-hexaméthyl-4,8-dioxa-1,11-undécanediol (CAS : 112548-49-9), du 1-phényl-1,11-undécanediol (CAS : 109217-58-5), du 2-octyl-1,11-undécanediol (CAS : 48074-21-1), du 2,10-diéthyl-2,10-diméthyl-1,11-undécanediol (CAS : 85018-66-2), du 2,2,10,10-tétramethyl-1,11-undécanediol (CAS : 35449-37-7), du 1-phényl-1,11-undécanediol (CAS : 109217-58-5), du 1,2-undécanediol (CAS : 13006-29-6), du 1,2-dodécanediol (CAS : 1119-87-5), du 2,11-dodécanediol (CAS : 33666-71-6), du 2,11-diéthyl-2,11-diméthyl-1,12-dodécanediol (CAS : 85018-68-4), du 2,11-diméthyl-2,11-dipropyl-1,12-dodécanediol (CAS : 85018-69-5), du 2,11-dibutyl-2,11-diméthyl-1,12-dodécanediol (CAS : 85018-70-8), du 2,2,11,11-tétraméthyl-1,12-dodécanediol (CAS : 5658-47-9), du 1,11-dodécanediol (CAS : 80158-99-2), du 11-méthyl-1,7-dodécanediol (CAS : 62870-49-9), du 1,4-dodécanediol (CAS : 38146-95-1), du 1,3-dodécanediol (CAS : 39516-24-0), du 1,10-dodécanediol (CAS : 39516-27-3), du 2,11-diméthyl-2,11-dodécanediol (CAS : 22092-59-7), du 1,5-dodécanediol (CAS : 20999-41-1), du 6,7-dodécanediol (CAS : 91635-53-9), et de leurs mélanges.

De préférence, le diol est choisi dans le groupe constitué de l'éthylène glycol (CAS : 107-21-1), du 1,6-hexanediol, du 3-méthyl-1,5-pentanediol (MPD, CAS : 4457-71-0), du 2,2-diméthyl-1,3-propanediol (Néopentylglycol - NPG, CAS : 126-30-7), et de leurs mélanges.

De préférence, le polyester de formule générale [Chem 7] est obtenu par réaction de polycondensation entre : l'acide adipique ; et un mélange du néopentyl glycol, de l'éthylène glycol, et du 1,6-hexanediol ;
ou entre : l'acide adipique ; et le 3-méthyl-1,5-pentanediol.

De préférence, le polyester diol de formule générale [Chem 7] a un indice d'hydroxyle $I_{OH}$ compris entre 4 et 24 mg KOH/g, préférentiellement entre 7 et 24 mg KOH/g, de préférence entre 7 et 20 mg KOH/g, et en particulier entre 9 et 19 mg KOH/g. De préférence, l'indice d'hydroxyle $I_{OH}$ est compris entre 9 et 24 mg KOH/g.

Le polyester diol de formule générale [Chem 7] peut avoir une température de transition vitreuse $T_g$ inférieure à 0°C, de préférence inférieure ou égale à -20°C, de préférence inférieure ou égale à -40°C, préférentiellement inférieure ou égale à -50°C, en particulier inférieure ou égale à -60°C, par exemple inférieure ou égale à -64°C.

Le polyester diol de formule générale [Chem 7] peut avoir une masse moléculaire moyenne en nombre supérieure ou égale à 5 500 g/mol, de préférence supérieure ou égale à 6 000 g/mol, en particulier strictement supérieure à 6 000 g/mol, préférentiellement supérieure ou égale à 8 000 g/mol, en particulier supérieure ou égale à 9 000 g/mol, par exemple supérieure ou égale à 10 000 g/mol, avantageusement supérieure ou égale 12 000 g/mol, et en particulier supérieure ou égale 18 000 g/mol.

La masse moléculaire moyenne en nombre du polyester diol de formule générale [Chem 7] peut être déterminée à partir de son $I_{OH}$ et de sa fonctionnalité.

Parmi les polyesters diols amorphes de formule générale [Chem 7], on peut par exemple citer le DYNACOLL ® 7250 commercialisé par EVONIK (polyester polyol ayant une viscosité de 180 Pa.s à 23°C, une masse moléculaire moyenne en nombre Mn égale à 5 500 g/mol, et une $T_g$ égale à - 50°C), le KURARAY ® P-6010 commercialisé par KURARAY (polyester polyol ayant une viscosité de 68 Pa.s à 23°C, une masse moléculaire moyenne en nombre égale à 6 000 g/mol, et une $T_g$ égale à -64°C), ou le KURARAY ® P-10010 commercialisé par KURARAY (polyester polyol ayant une viscosité de 687 Pa.s à 23°C, une masse moléculaire moyenne en nombre égale à 10 000 g/mol).

La deuxième étape du procédé d'obtention du second polymère de formule générale [Chem 5] spécifique consiste en la préparation du polymère de formule générale [Chem 5].

Selon une première variante, le(s) polyester(s) de formule générale [Chem 7] susmentionné(s) peu(ven)t être mis en réaction avec l'isocyanatosilane de formule générale [Chem 20]

$$NCO\text{-}R^3\text{-}Si(R^4)_p(OR^5)_{3-p}$$

à raison d'une quantité correspondant à un rapport équivalent molaire des nombres de fonctions NCO/OH comprise entre 0,90 et 1,05 et de préférence égal à environ 1.

Cette étape est en particulier conduite dans des conditions anhydres, de manière à éviter l'hydrolyse des groupes alkoxysilanes. Un domaine de température typique pour la mise en œuvre de cette étape réactionnelle est de 30°C à 120°C, et plus particulièrement de 60°C à 105°C.

Les isocyanatosilanes de formule générale [Chem 20] susmentionnée sont largement disponibles dans le commerce. On peut notamment citer le SILQUEST ® A-LINK 35 soit le (3-isocyanatopropyl)triméthoxysilane) disponible auprès de MOMENTIVE, le SILQUEST ® A-LINK 25 soit le (3-isocyanatopropyl)triéthoxysilane) disponible auprès de MOMENTIVE, le (3-isocyanatopropyl)méthyldiméthoxysilane disponible auprès de GELEST, le GENIOSIL ® XL 42 soit le (3-isocyanatométhyl)méthyldiméthoxysilane disponible auprès de WACKER et le GENIOSIL ® XL 43 soit le (3-isocyanatométhyl)triméthoxysilane disponible auprès de WACKER. Selon une seconde variante, le polymère silylé peut être obtenu en deux étapes par :

réaction du(des) polyester polyol(s) de formule générale [Chem 7] avec un diisocyanate de formule $NCO\text{-}R^1\text{-}NCO$ dans des quantités correspondant à un rapport équivalent molaire des nombres de fonctions NCO/OH compris entre 0,3 et 0,7 et de préférence égal à environ 0,5, pour former un bloc polyester-polyuréthane ;
réaction entre un bloc obtenu à l'étape précédente avec l'isocyanatosilane de formule [Chem 20], à raison d'une quantité correspondant à un rapport équivalent molaire des nombres de fonctions NCO/OH comprise entre 0,90 et 1,05 et de préférence égal à environ 1.

Les isocyanatosilanes peuvent être ceux mentionnés ci-dessus.

De préférence, le polymère silylé selon l'invention est un polymère de formule générale [Chem 3] dans laquelle :

$m_1$ est un nombre entier égal à 0 ;

$$p = 1 ;$$

$R^4$ et $R^5$ représentent chacun un radical méthyle ;

$R^3$ représente un radical divalent méthylène ;

la masse moléculaire moyenne en nombre dudit polymère va de 5 000 à 30 000 g/mol, préférentiellement de 10 000 à 20 000 g/mol, en particulier de 14 000 g/mol à 15 000 g/mol.

De préférence, le polymère silylé selon l'invention est un polymère de formule générale [Chem 3] dans laquelle :

$m_1$ est un nombre entier différent de 0 ;

$$p = 0 ;$$

$R^3$ représente un radical divalent propylène ;
$R^5$ représente un radical méthyle ;

la masse moléculaire moyenne en nombre dudit polymère va de 5 000 à 30 000 g/mol, préférentiellement de 10 000 à 30 000 g/mol, en particulier de 15 000 à 25 000 g/mol.

De préférence, le polymère silylé selon l'invention est un polymère de formule générale [Chem 5] dans laquelle :

q est un nombre entier différent de 0 ;

$$p = 0 ;$$

$R^3$ représente un radical divalent propylène ;
$R^5$ représente un radical méthyle ;
la masse moléculaire moyenne en nombre dudit polymère va de 5 000 à 30 000 g/mol, préférentiellement de 10 000 à 30 000 g/mol, en particulier de 15 000 à 25 000 g/mol.

La composition adhésive peut comprendre un seul polymère tel que décrit ci-dessus.
Alternativement, la composition adhésive peut comprendre plus d'un polymère silylé, par exemple deux, ou trois, ou quatre, ou cinq, ou plus que cinq polymères silylés.
La composition adhésive selon l'invention peut comprendre de 20 à 90 %, de préférence de 30 à 70 % en poids de polymère silylé, par rapport au poids total de la composition adhésive.

Composé polyvinyléther

Le composé polyvinyléther selon l'invention est un homopolymère choisi parmi le poly(méthylvinyléther), le poly(éthylvinyléther), le poly(butylvinyléther), le poly(isobutylvinyléther), le poly(isopropylvinyléther), le poly(propylvinyléther), le poly(octylvinyléther) et leurs mélanges.
Le composé polyvinyléther selon l'invention peut présenter une valeur K de 30 à 120, et de préférence de 40 à 70. Par « *valeur K* » on entend une mesure du degré moyen de polymérisation. La valeur K est mesurée selon la norme ISO 1628-1.
Le composé polyvinyléther selon l'invention peut en outre présenter une température de transition vitreuse de -60 à 0°C, et de préférence de -50 à - 5°C. La température de transition vitreuse a été mesurée par calorimétrie différentielle à balayage (DSC). La température de transition vitreuse peut être mesurée selon la norme NF EN ISO 11357-2.
Des composés polyvinyléther commerciaux peuvent inclure les Lutonal ® M 40, Lutonal ® A 25, Lutonal ® A 50, Lutonal ® A 100, Lutonal ® I 30, Lutonal ® I 60, Lutonal ® I 60 D, Lutonal ® I 65 D disponibles auprès de la société BASF et le Gantrez ® M disponible auprès de la société GAF.
La composition adhésive selon l'invention peut comprendre de 1 à 60 %, de préférence de 5 à 40 % et encore de préférence de 5 à 25 % en poids de composé polyvinyléther, par rapport au poids total de la composition adhésive.

Catalyseur de réticulation

Le catalyseur de la composition adhésive peut être choisi dans le groupe constitué des amines, des composés organométalliques, des acides et de leurs dérivés, et de leurs mélanges. Il peut s'agir d'un mélange de catalyseurs d'une même famille (par exemple un mélange de plusieurs amines), ou un mélange de catalyseurs de différentes familles (par exemple un mélange d'une amine et d'un composé organométallique).
Dans le cadre de l'invention, on entend par « *composés organométalliques »,* des composés comprenant un radical organique et au moins un métal.
Dans le cadre de l'invention, on entend par « *radical organique »,* un radical comprenant au moins un atome de carbone.
Les composés organométalliques peuvent comprendre les composés organométalliques (composés comprenant au moins une liaison covalente métal-carbone), les alcoolates métalliques, les carboxylates métalliques, et les complexes de coordination métalliques avec un (des) ligand(s) organique(s).
A titre d'exemple de ligand organique, on peut citer l'acétylacétonate et les oximes.
L'atome métallique des composés organométalliques peut être tout atome métallique connu de l'homme du métier, et peut en particulier être choisi parmi l'étain, l'aluminium, le zinc, le cobalt, le fer, le nickel, le bismuth, le titane, ou le zirconium. Les composés organométalliques peuvent d'ailleurs comprendre plusieurs atomes métalliques.
Les composés organométalliques (composés comprenant au moins une liaison covalente métal-carbone) peuvent être des carboxylates de composés organométalliques.
Les composés organométalliques peuvent être choisis dans le groupe constitué du dilaurate de dibutyle étain

(DBTL), du diacétate de dibutyle étain, du diéthylhexanoate de dibutyle étain, du dinéodécanoate de dioctyle étain (par exemple disponible sous la dénomination TIB KAT ® 223 auprès de la société TIB CHEMICALS), du dioléate de dibutyle étain, du benzylmaléate de dibutyle étain, du diacétate de diphényle étain, et de leurs mélanges.

Les alcoolates métalliques peuvent être choisis dans le groupe constitué du tétrabutanolate de titane, du tétraisopropylate de titane, du tétrabutanolate de zirconium, du tétratisopropylate de zirconium, et de leurs mélanges.

Les carboxylates métalliques peuvent être choisis dans le groupe constitué du 2-éthylcaproate de zinc, du diacétate de zinc, du dinéodécanoate de zinc, du diundécénoate de zinc, du diméthacrylate de zinc, de l'acétylacétonate de cobalt, du diacétate de cobalt, de l'acétylacétonate de fer, du diacétate de fer, de l'acétylacétonate de nickel, du diacétate de nickel, de l'acétate de bismuth, du trioctanoate de bismuth, du dinéodécanoate de bismuth, du dinéodécanoate de zinc et de bismuth, et de leurs mélanges.

Les complexes de coordination métalliques avec un(des) ligand(s) organique(s) peuvent être choisis dans le groupe constitué de l'acétylacétonate de zinc, de l'acétylacétonate de titane (par exemple disponible commercialement sous la dénomination TYZOR ® AA75 auprès de la société DORF KETAL), du tétraacétylacétonate de titane, du trisacétylacétonate d'aluminium, des chélates d'aluminium tel que par exemple le mono-acétylacetonate bis(éthylacétoacétate) (par exemple disponible commercialement sous la dénomination K-KAT ® 5218 auprès de la société KING INDUSTRIES), du tétraacétylacétonate de zirconium, du diisopropoxybis(éthylacétonato)titane, et de leurs mélanges.

Les amines peuvent être des amines primaires, des amines secondaires ou des amines tertiaires.

Les amines peuvent être des aminosilanes, tels que par exemple l'aminopropyltriméthoxysilane, le N-(2-aminoéthyl)-3-aminopropyltriméthoxysilane, l'aminopropyltriéthoxysilane, le N-(2-aminoéthyl)-3-aminopropyltriéthoxysilane, le bis(gamma-triméthoxysilylpropyl)amine, le N-éthyl-gamma-aminoisobutyltriméthoxysilane, ou le N-phénylgamma-aminopropyltriméthoxysilane.

De préférence, le catalyseur n'est pas un aminosilane.

De préférence, les amines sont choisies dans le groupe constitué de la triéthylamine, de la tributylamine, de la tétraméthylguanidine, du 1,8-diazabicyclo[5.4.0]-7-undécène, du 1,4-diazabicyclo[2.2.2]octane, du 1,5-diazabicyclo [4.3.0]non-5-ène, de la N,N-bis(N,N-diméthyl-2-aminoéthyl)méthylamine, de la N,N-diméthylcyclohexaylamine, de la N,N-diméthylphénylamine, de la N-éthylmorpholine, et de leurs mélanges.

Les catalyseurs acides peuvent être choisis parmi les catalyseurs acides organiques, les catalyseurs acides inorganiques, et leurs mélanges.

Parmi les catalyseurs acides inorganiques, on peut par exemple citer l'acide phosphorique ou orthophosphorique, l'acide phosphoreux, l'acide hypophosphoreux, ou l'acide sulfurique.

De préférence, les catalyseurs acides organiques ont un pKa inférieur ou égal à 6, de préférence inférieur ou égal à 4, avantageusement inférieur ou égal à 2, avantageusement inférieur ou égal à 0.

Les catalyseurs acides organiques peuvent être choisis parmi les acides sulfoniques, les acides carboxyliques, les organophosphates acides, les organophosphonates acides, les acides phosphoniques, et leurs mélanges. Les acides sulfoniques peuvent être aliphatiques ou aromatiques, éventuellement substitués (par exemple substitués par au moins un substituant choisi parmi les halogènes (tels que le fluor), les hydroxyles, les alkyles, les amines, et leurs mélanges), et peuvent être mono- ou disulfoniques.

Les acides sulfoniques peuvent être choisis parmi les acides N-alkylaminoalkylsulfoniques et les acides N,N-dialkylaminoalkylsulfoniques (zwitterions), tels que par exemple l'acide 2-(N-morpholino)éthanesulfonique, l'acide 3-(N-morpholino)propanesulfonique, l'acide 4-[N-morpholino]butanesulfonique, l'acide 1,4 -pipérazinediéthanesulfonique, l'acide N-2-hydroxyéthylpipérazine-N'-2-éthanesulfonique, l'acide 2-(N-morpholino)éthanesulfonique, l'acide N-morpholinométhanesulfonique, l'acide N-(2-hydroxyéthyl)pipérazine-N'-méthanesulfonique, l'acide pipérazine-N,N'-bis(méthanesulfonic), l'acide cyclohexylaminométhanesulfonique, l'acide N-[tris(hydroxyméthyl)méthyl]-aminométhanesulfonique, l'acide N,N-bis(2-hydroxyéthyl)aminométhanesulfonique; l'acide para-toluène sulfonique ; l'acide benzène sulfonique ; l'acide méthanesulfonique ; l'acide dodécylbenzène disulfonique ; l'acide dodécylbenzène sulfonique ; l'acide dinonylnaphtalène disulfonique; l'acide dinonylnaphtalène sulfonique ; l'acide trifluorométhylsulfonique ; et leurs mélanges.

En particulier, les acides sulfoniques sont choisis parmi l'acide para-toluène sulfonique, l'acide benzène sulfonique, l'acide méthanesulfonique, l'acide dodécylbenzène sulfonique, l'acide dodécylbenzène disulfonique, l'acide dinonylnaphtalène disulfonique, l'acide dinonylnaphtalène sulfonique, l'acide trifluorométhylsulfonique, et leurs mélanges.

Parmi les catalyseurs acides carboxyliques, on peut par exemple citer l'acide malonique, l'acide succinique, l'acide maléïque, l'acide oxalique, l'acide acétique, l'acide lactique, l'acide benzoïque, l'acide citrique, l'acide glycolique, et leurs mélanges.

Dans le cadre de l'invention, et sauf mention contraire, on entend par « *organophosphate acide* », un ester de l'acide phosphorique comprenant au moins un radical -OH. Par exemple, le phosphate de méthyle est un organophosphate acide comprenant deux radicaux -OH et de formule générale [Chem 22]

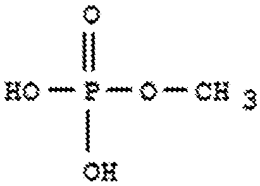

En particulier, les organophosphates acides ont la formule générale

[Chem 23] $(R^{10}O)_g$-(P=O)-$(OH)_h$

dans laquelle :

$R^{10}$ est un radical organique, en particulier un radical choisi parmi les alkyles en C1-C22, linéaire ou ramifiés, les cycloalkyles, les aryles, et leurs mélanges (lesdits groupes alkyles, cycloalkyles et aryles étant éventuellement substitués) ; et

g et h sont des nombres entiers, avec g + h = 3 et h = 1 ou 2.

Les organophosphates acides, peuvent par exemple être choisis dans le groupe constitué des mono- ou dialkyle phosphates acides en C1-C22 et de leurs mélanges, tel que par exemple le phosphate de butyle, le phosphate de dibutyle, le phosphate de di-(2-éthylhexyle), le phosphate de 2-éthylhexyle et leurs mélanges ; des phosphates de mono-, ou diaryle, et de leurs mélanges, tel que par exemple le phosphate de monophényle, le phosphate de diphényle et leurs mélanges ; des alkyle-phényle phosphates ; et de leurs mélanges.

Dans le cadre de l'invention, et sauf mention contraire, on entend par « organophosphonate acide », un composé phosphoré ayant la formule générale [Chem 24]

$R^{11}$-(P=O)-(OH)$(OR^{12})$

dans laquelle $R^{11}$ et $R^{12}$ sont des radicaux organiques, de préférence choisis indépendamment l'un de l'autre, parmi les alkyles en C1-C22, linéaire ou ramifiés, les cycloalkyles, les aryles, et leurs mélanges (lesdits groupes alkyles, cycloalkyles et aryles étant éventuellement substitués).

Parmi les organophosphonates acides, on peut par exemple citer les mono alkyle phosphonates acides en C1-C22.

Dans le cadre de l'invention, et sauf mention contraire, on entend par « acide phosphonique », un composé phosphoré ayant la formule générale [Chem 25]

$R^{13}$-(P=O)-$(OH)_2$

dans laquelle $R^{13}$ est un radical organique, de préférence choisi parmi les alkyles en C1-C22, linéaire ou ramifiés, les cycloalkyles, les aryles, et leurs mélanges (lesdits groupes alkyles, cycloalkyles et aryles étant éventuellement substitués).

Parmi les acides phophoniques, on peut par exemple citer les acides N-alkylaminoalkylphosphoniques (zwitterions), les acides N,N-dialkylaminoalkylphosphoniques (zwitterions), les alkylphosphoniques en C1-C20 tels que par exemple l'acide méthylphosphonique, l'acide ethyl phosphonique, l'acide propylphosphonique, l'acide butylphosphonique, l'acide t-butylphosphonique, l'acide isobutylphosphonique, l'acide hexylphosphonique, l'acide ethyl-2-hexylphosphonique et homologues supérieurs linéaires ou ramifiés, l'acide benzylphosphonique, l'acide phénylphosphonique, l'acide toluylphosphonique, l'acide xylylphosphonique. A titre de catalyseurs acides organiques, on peut par exemple citer le NACURE ® 155 (acide dinonylnaphtalène disulfonique, à 55 % de matière active dans l'isobutanol) commercialisé par KING INDUSTRIES, le NACURE ® 1051 (acide dinonylnaphtalène sulfonique, à 50 % de matière active dans le 2-butoxyéthanol) commercialisé par KING INDUSTRIES, le NACURE ® 5076 (acide dodécylbenzène sulfonique, à 70% de matière active dans l'isopropanol) commercialisé par KING INDUSTRIES, le K-CURE ® 1040 (acide para-toluène sulfonique, à 40 % de matière active dans l'isopropanol) commercialisé par KING INDUSTRIES, le NACURE ® 4000 (mélange de mono et dialkyle phosphates acides, 100 % de matière active) commercialisé par KING INDUSTRIES.

Les dérivés d'acide selon l'invention, peuvent être des anhydrides d'acide, des esters d'acide, des sels d'ammonium d'acide, l'acide étant tel que décrit ci-dessus.

Les dérivés d'acide sont en particulier des acides dits « *bloqués* » ou encore « *latents* » qui permettent avantageusement de libérer l'acide par activation thermique (par exemple à une température allant de 70°C à 170°C, de préférence à une température allant de 90°C à 120°C) ou par hydrolyse, ou par photoactivation, de préférence par

activation thermique.

L'acide bloqué permet avantageusement de libérer l'acide qui est l'entité ayant l'activité catalytique. Par exemple, le sel d'ammonium formé entre l'amino méthyl propanol et l'acide para-toluène sulfonique est un acide bloqué (dérivé d'acide) qui par activation thermique libère l'acide para-toluène sulfonique.

Les dérivés d'acide peuvent être préparés par tout moyen connu de l'homme du métier à partir de l'acide correspondant, par exemple par utilisation des réactions typiques acides/bases. Par exemple, le procédé pour faire un ester implique typiquement la condensation d'un composé acide avec un composé comprenant un groupe hydroxyle tel que par exemple un alcool, ou avec un composé de type oxirane. Les sels d'ammonium peuvent être préparés à partir de tout acide susmentionné, avec l'ammoniaque ou avec une amine primaire, secondaire ou tertiaire. Les amines peuvent éventuellement comprendre au moins un groupe fonctionnel tel qu'un groupe hydroxy (alcanolamines), un groupe alkyle en C1-C4. Les sels d'ammonium (zwitterions) peuvent également être préparés par modification du pH d'une solution contenant par exemple des acides N-alkylaminoalkylphosphoniques, des acides N,N-dialkylaminoalkylphosphoniques, des acides N-alkylaminoalkylsulfoniques ou encore des acides N,N-dialkylaminoalkylsulfoniques.

De préférence, le catalyseur est un sel d'ammonium d'un acide sulfonique (l'acide sulfonique étant tel que décrit ci-dessus), un sel d'ammonium d'un acide phosphonique (l'acide phosphonique étant tel que décrit ci-dessus), un sel d'ammonium d'un organophosphonate acide (l'organophosphonate acide étant tel que décrit ci-dessus), ou un sel d'ammonium d'un organophosphate acide (organophosphate acide étant tel que décrit ci-dessus).

A titre d'amines pour la préparation des sels d'ammonium, on peut par exemple citer le 2-amino-2-méthyl-1-propanol, la triéthylamine, l'aniline, la pyridine, le diméthylaminoéthanol, les alkypyridines, la diisopropanolamine, la diméthyléthanolamine, la triéthanolamine, les oxazolidines, les oxazolidines bicycliques, les amidines, les diazabicyclooctanes, les guanidines, les N-alkyl morpholine, les aminopyridine, les aminoalkylpyridines, les aminopyrrolidines, l'indazole, l'imidazole, le pyrazole, la pyrazine, la pyrimidine, la purine, l'imidazoline, la pyrazoline, la pipérazine, l'aminomorpholine, les aminoalkylmorpholines, et leurs mélanges. De préférence, les amines sont des amines tertiaires.

A titre de dérivés d'acide, on peut par exemple citer le NACURE ® 3327 ou le NACURE ® 3525 (acide dinonylnaphtalène disulfonique bloqué par une amine, à 25 % de matière active dans l'isopropanol et l'isobutanol) commercialisé par KING INDUSTRIES, le NACURE ® 1557 ou le NACURE ® 1953 (acide dinonylnaphtalène sulfonique bloqué par une amine, à 25 % de matière active dans un mélange butanol et 2-butoxyéthanol) commercialisé par KING INDUSTRIES, le NACURE ® 5225 ou NACURE ® 5528 ou NACURE ® 5925 (acide dodécylbenzène sulfonique bloqué par une amine, à 25 % de matière active dans l'isopropanol) commercialisé par KING INDUSTRIES, le NACURE ® 2107 ou NACURE ® 2500 (acide para-toluène sulfonique bloqué par une amine, à 25 % ou 26 % de matière active dans l'isopropanol) commercialisé par KING INDUSTRIES, le NACURE ® 2501 ou NACURE ® 2530 (acide para-toluène sulfonique bloqué par une amine, à 25 % de matière active dansun mélange isopropanol et méthanol) commercialisé par KING INDUSTRIES, le NACURE ® 4167 (dialkyle phosphate bloqué par une amine organique, à 25% de matière active dans un mélange isopropanol et isobutanol) commercialisé par KING INDUSTRIES, le NACURE ® 4575 (phosphate acide bloqué par une amine, à 25 % de matière active dans un mélange 30 méthanol et butanol) commercialisé par KING INDUSTRIES.

De préférence, le catalyseur est choisi dans le groupe constitué des composés organométalliques (en particulier les complexes de coordinations à base d'aluminium), de l'acide orthophosphorique, des organophosphates acides (de préférence mono- ou dialkyle phosphate acide en C1-C22 et leurs mélanges), des sels d'ammonium (en particulier d'acide sulfonique ou d'organophosphate acide), et de leurs mélanges. De façon encore plus préférée, le catalyseur est choisi dans le groupe constitué de l'acide orthophosphorique, des organophosphates acides (de préférence mono- ou dialkyle phosphate acide en C1-C22 et leurs mélanges), des sels d'ammonium (en particulier d'acide sulfonique ou d'organophosphate acide). La composition adhésive selon l'invention peut comprendre de 0,1 à 4 %, de préférence de 0,2 à 2 % et encore de préférence de 0,3 à 2 % en poids de catalyseur de réticulation, par rapport au poids total de la composition adhésive.

Dans le cas où la composition selon l'invention est une composition mono-composante, il est préférable d'utiliser un des catalyseurs cités ci-dessus autre que les acides inorganiques et leurs sels, et aussi autre que les acides carboxyliques.

Dans le cas où la composition selon l'invention est une composition bi-composante (comme détaillé ci-dessous) le catalyseur de réticulation peut être mélangé dans un diluant réactif ou non réactif. Par « diluant non réactif » on entend un diluant qui ne réagit pas en présence du catalyseur. Ainsi, par « diluant réactif » on entend un diluant qui réagit en présence du catalyseur. Le diluant peut être choisi parmi des monosilanes faiblement réactifs (Geniosil XM25, MS RD359), des water scavengers (XL33) et des résines silsesquioxanes,

Non réactif : des polyols, des résines tackifiantes, les modifiants rhéologiques (cités ds autres additifs), ...

Ce diluant peut avoir une masse moléculaire en nombre (Mn) allant de 300 à 50 000 g/mol et de préférence 1 000 à 20 000 g/mol. Ceci permet d'éviter la migration de ce diluant dans l'adhésif réticulé.

Ce diluant peut également avoir une viscosité allant de 10 à 100 000 mPa.s à 23°C et encore plus préférentiellement de 500 à 15 000 mPa.s à 23°C. Cette viscosité est mesurée avec un viscosimètre Brookfield (cône plan,

CAP2000+).

Autres additifs

La composition selon la présente invention peut comprendre au moins un autre additif, par exemple choisi dans le groupe constitué des résines tackifiantes, silsequioxanes, des plastifiants, des solvants, des pigments, des colorants, des promoteurs d'adhérence, des absorbeurs d'humidité, des stabilisants UV, des antioxydants, des paillettes, des matériaux fluorescents, des additifs rhéologiques, des charges, des agents ignifugeants, des cires, et de leurs mélanges.

Résine tackifiante

La composition adhésive selon l'invention peut comprendre en outre au moins une résine tackifiante.

La ou les résines utilisées dans le cadre de l'invention peut(peuvent) être toute(s) résine(s) compatible(s) avec le(s) polymère(s) silylé(s).

Par « *résine tackifiante compatible* », on entend une résine tackifiante qui, lorsqu'elle est mélangée dans les proportions 50/50 en poids avec le(s) polymère(s) silylé(s), donne un mélange substantiellement homogène (pas de déphasage observé visuellement). Dans le cadre de l'invention, la résine tackifiante est différente du composé polyvinyléther.

Les résines tackifiantes sont avantageusement choisies parmi :

- les résines terpènes-phénoliques ;
- les résines d'hydrocarbures ;
- les résines de colophane ; et
- les résines acryliques.

Les résines terpènes-phénoliques peuvent avoir un point de ramollissement de 85 à 150°C. Les résines d'hydrocarbures peuvent avoir un point de ramollissement de 15 à 140°C. Les résines de colophane peuvent avoir un point de ramollissement de 15 à 115°C. Le point de ramollissement peut être mesuré selon la norme ASTM E28 standard.

Les résines terpènes-phénoliques peuvent être obtenues par polymérisation d'hydrocarbures terpéniques et de phénols, en présence de catalyseur(s) de Friedel-Crafts.

Les résines d'hydrocarbures peuvent être choisies parmi : les résines obtenues par un procédé comprenant la polymérisation d'alpha-méthyl styrène, et éventuellement en présence de phénols ; les résines obtenues par hydrogénation, préférentiellement hydrogénation partielle, polymérisation ou copolymérisation (avec un hydrocarbure aromatique) d'un mélange d'hydrocarbures aliphatiques insaturés ayant environ 5, 9 ou 10 atomes de carbone dérivés des fractions de pétrole, optionnellement greffés avec de l'anhydride maléique ; les résines terpéniques ; généralement résultant de la polymérisation d'hydrocarbures terpéniques tels que par exemple le monoterpène (ou pinène) en présence de catalyseur(s) de Friedel-Crafts ; les copolymères à base de terpènes naturels, tels que par exemple le styrène/terpène, l'alpha-méthyl styrène/terpène et le vinyl toluène/terpène ; et leurs mélanges.

Les résines de colophane peuvent être choisies parmi les colophanes d'origine naturelle ou modifiées (telles que par exemple la colophane extraite de la gomme de pins, la colophane de bois extraite des racines de l'arbre) et leurs dérivés hydrogénés, dimérisés, polymérisés ou estérifiés par des monoalcools ou des polyols (comme par exemple le glycérol ou le pentaérythritol).

Une résine acrylique est définie comme un polymère ou un oligomère construit avec une quantité significative de monomères (méth)acrylique et/ou (méth)acrylate, de préférence au moins 5% poids/poids (w/w), plus préférablement au moins 10% w/w, encore plus préférablement au moins 20% w/w, encore plus préférablement au moins 30% w/w dans la chaîne polymère.

Les monomères (méth)acryliques peuvent être choisis parmi les : acide acrylique, acide méthacrylique, acrylate de méthyle, méthacrylate de méthyle, acrylate d'éthyle, méthacrylate d'éthyle, acrylate de butyle, méthacrylate de butyle, acrylate d'isobutyle, méthacrylate d'isobutyle, acrylate de n-hexyle, méthacrylate de n-hexyle, acrylate de 2-éthylhexyle, méthacrylate de 2-éthylhexyle, acrylate de n-heptyle, méthacrylate n-heptyle, acrylate de stéaryle, méthacrylate de stéaryle, méthacrylate de glycidyle, crotonates d'alkyle, acétate de vinyle, maléate de di-n-butyle, di-octylmaléate, méthacrylate d'acétoacétoxyéthyle, acrylate d'acétoacétoxyéthyle, méthacrylate d'acétoacétoxypropyle, acrylate d'acétoacétoxypropyle, diacétone acrylamide, acrylamide, méthacrylamide, méthacrylate de hydroxyéthyle, acrylate de hydroxyéthyle, méthacrylate d'allyle, méthacrylate de tetrahydrofurfuryl, acrylate de tetrahydrofurfuryl, cyclohexylméthacrylate, acrylate de cyclohexyle, acrylate de n-hexyle, méthacrylate de n-hexyle, acrylate de 2-éthoxyéthyle, méthacrylate de 2-éthoxyéthyle, méthacrylate d'isodecyle, acrylate d'isodecyle, 2-méthoxy acrylate, 2-méthoxy méthacrylate, 2-(2-éthoxyéthoxy)éthylacrylate, 2-phenoxyéthyle acrylate, 2-phenoxyéthyle méthacrylate, acrylate d'isobornyle, le

méthacrylate d'isobornyle, acrylate de caprolactone, méthacrylate de caprolactone, monométhacrylate de polypropylèneglycole, monoacrylate de polypropylèneglycole, acrylate de polyéthylèneglycole (400), méthacrylate de polypropylèneglycole (400), acrylate de benzyle, benzylméthacrylate, N-vinyle pyrrolidone ou N-vinyle lactame.

De préférence, les monomères (méth)acryliques ont jusqu'à 20 atomes de carbone, plus préférentiellement, les monomères (méth)acryliques sont choisis parmi l'acide acrylique, l'acide méthacrylique, l'acrylate de butyle, l'acrylate de 2-éthylhexyle et l'hydroxyéthylacrylate.

Les résines acryliques peut être choisies parmi les polymères contenant au moins une fonction ou une partie de chaîne (méth)acrylique et au moins une partie de chaîne hydrocarbonée, lesdits polymères pouvant se présenter sous forme de copolymères, greffés ou réagis ou séquencés.

Les résines acryliques ont de préférence une viscosité à 100°C inférieure à 100 Pa.s et inférieur ou égal à 100 Pa.s à 150°C.

Les résines acryliques peuvent comprendre des unités répétitives d'au moins un monomère hydrocarboné et d'au moins un monomère acrylate.

Les monomères hydrocarbonés sont choisis dans le groupe constitué de styrène, alpha-méthyl styrène, vinyle toluène, indène, méthylindène, divinylbenzène, dicyclopentadiène et méthyl-dicyclopentadiène, et des monomères polymérisables contenus dans les courants C5-pyperyléniques et C5-isoprène et C9-aromatiques de l'industrie pétrochimique. Ces monomères hydrocarbonés sont habituellement polymérisés ensemble dans divers rapports par polymérisation cationique en utilisant des catalyseurs d'acide de Lewis.

Les monomères acrylates sont choisis parmi l'acrylate de méthyle, l'acide acrylique, l'acide méthacrylique, le méthacrylate de méthyle, l'acrylate d'éthyle, le méthacrylate d'éthyle, l'acrylate de butyle, le méthacrylate de butyle, l'acrylate d'isobutyle, le méthacrylate d'isobutyle, l'acrylate de n-hexyle, l'acrylate de n-hexylméthacrylate, l'éthylhexyle, le méthacrylate d'éthylhexyle, l'acrylate de n-heptyle, le méthacrylate d'heptyle, le 2-méthyle (méth)acrylate d'heptyle, l'acrylate d'octyle, le méthacrylate d'octyle, le (méth) acrylate de isooctyle, le (méth) acrylate de n-nonyle, le (méth) acrylate d'iso-nonyle, le (méth) acrylate de décyle, l'acrylate d'isodécyle, le méthacrylate d'isodécyle, le (méth)acrylate de dodécyle, le (méth)acrylate d'isobornyle, le méthacrylate de lauryle, l'acrylate de lauryle, l'acrylate de tridécyle, le méthacrylate de tridécyle, l'acrylate de stéaryle, le méthacrylate de stéaryle, le méthacrylate de glycidyle, les crotonates d'alkyle, l'acétate de vinyle, le di-n-butylmaléate, le di-octylmaléate, le méthacrylate d'acétoacétoxyéthyle, l'acrylate d'acétoacétoxyéthyle, le méthacrylate d'acétoacétoxypropyle, l'acrylate d'acétoacétoxypropyle, le diacétone acrylamide, l'acrylamide, le méthacrylamide, le hydroxyéthylméthacrylate, le hydroxyéthylacrylate, le méthacrylate d'allyle, le méthacrylate de tétrahydrofurfuryle, l'acrylate de tétrahydrofurfuryle, le méthacrylate de cyclohexyle, l'acrylate de cyclohexyle, l'acrylate de n-hexyle, le méthacrylate de n-hexyle, l'acrylate de 2-éthoxyéthyle, le méthacrylate de 2-éthoxyéthyle, le méthacrylate d'isodécyle, l'acrylate d'isodécyle, l'acrylate de 2-méthoxy, le méthacrylate de 2-méthoxy, l'acrylate de 2-(2-éthoxyéthoxy) éthyle, l'acrylate de 2-phénoxyéthyle, le méthacrylate de 2-phénoxyéthyle, l'acrylate d'isobornyle, le méthacrylate d'isobornyle, l'acrylate de caprolactone, le méthacrylate de caprolactone, le monoacrylate de polypropylènegycole, le monométhacrylate de polypropylènegycole, l'acrylate de poyéthylèneglycol (400), le méthacrylate de polypropylèneglycol (400), l'acrylate de benzyle acrylate, le méthacrylate de benzyle, le sulfonate de sodium 1-allyloxy-2-hydroylpropyl, l'acrylonitrile et leurs mélanges.

De préférence, les monomères hydrocarbonés sont choisis dans le groupe des monomères aromatiques ou des monomères polymérisables du courant aromatique en C9 provenant de sources pétrochimiques ; de dicyclopentadiène ou de monomères polymérisables provenant du courant de C5-pyperylène ou C5-isoprène provenant de sources pétrochimiques.

De préférence, les monomères acrylate sont choisis parmi l'acide acrylique l'acrylate de 2-éthylhexyle, l'hydroxyéthylacrylate, l'acide méthacrylique, l'acrylate de butyle.

Le point de ramollissement de ces résines acryliques est de préférence de la température ambiante jusqu'à 180°C, de préférence inférieur ou égal à 150°C, plus préférablement inférieur ou égal à 120°C, et encore plus préférablement de 70 à 120°C. Le point de ramollissement peut être mesuré selon la norme ASTM E28 standard.

De telles résines sont disponibles commercialement, et on peut citer par exemple les produits suivants :
Pour les résines obtenues par polymérisation d'hydrocarbures terpéniques et de phénols, en présence de catalyseur(s) de Friedel-Crafts:

DERTOPHENE® 1510 disponible auprès de la société DRT possédant une masse moyenne moléculaire en nombre $M_n$ d'environ 870 Da ;
DERTOPHENE® H150 disponible auprès de la société DRT possédant une masse moyenne moléculaire en nombre $M_n$ d'environ 630 Da;
DERTOPHENE® T disponible auprès de la société DRT possédant une masse moyenne moléculaire en nombre $M_n$ d'environ 500 Da ;
SYLVAREZ® TP 95 disponible auprès de la société ARIZONA CHEMICAL ayant une masse moléculaire moyenne en nombre d'environ 1200 Da.

Pour les résines obtenues par un procédé comprenant la polymérisation d'alpha-méthyl styrène, et éventuellement en présence de phénols : CLEARTACK® W100 disponible auprès de la société CRAY VALLEY, obtenue par polymérisation d'alpha-méthyl styrène sans action de phénols, ayant une masse moléculaire moyenne en nombre de 900 Da ; SYLVAREZ® 510 qui est disponible auprès de la société ARIZONA CHEMICAL ayant une masse moléculaire moyenne en nombre d'environ 1740 Da, dont le procédé d'obtention comprend l'ajout de phénols.

Pour les colophanes d'origine naturelle ou modifiées : SYLVALITE® RE 100 qui est un ester de colophane et de pentaérythritol disponible auprès de la société ARIZONA CHEMICAL, et dont la masse moléculaire moyenne en nombre est d'environ 1700 Da.

Selon un mode de réalisation préféré, la résine tackifiante est choisie parmi les résines obtenues par polymérisation d'hydrocarbures terpéniques et de phénols, en présence de catalyseur(s) de Friedel-Crafts.

La résine tackifiante présente de préférence une masse moléculaire moyenne en nombre allant de 100 à 6 000 g/mol, de préférence de 300 à 4 000 g/mol, préférentiellement de 500 à 2 000 g/mol.

Les masses moléculaires moyennes en nombre des résines tackifiantes peuvent être mesurées selon des méthodes bien connues de l'homme du métier, par exemple par chromatographie d'exclusion stérique en utilisant un étalon de type polystyrène.

La résine tackifiante peut avoir un indice hydroxyle $I_{OH}$ allant de 10 à 300 mg KOH/g, de préférence allant de 100 à 200 mg KOH/g, préférentiellement allant de 140 à 160 mg KOH/g. En particulier, la résine tackifiante a un indice hydroxyle valant 145 mg KOH/g.

L'indice hydroxyle de la résine tackifiante représente le nombre de fonctions hydroxyles par gramme de résine tackifiante, et est exprimée sous la forme du nombre équivalent de milligrammes de potasse par gramme de résine tackifiante (mg KOH/g) pour le dosage des fonctions hydroxyles.

L'indice d'acide de le résine tackifiante type rosin ester peut aller de 0 à 10 mg KOH/g, et préférentiellement de 0 à 5 mg KOH/g. L'indice d'acide de la résine tackifiante représente le nombre de fonction acide par gramme de résine tackifiante, et est exprimé sous la forme de nombre équivalent de milligrammes de potasse par gramme de résine tackifiante mg KOH/g pour le dosage des fonctioncs acides

La composition adhésive selon l'invention peut comprendre de 0,1 à 80 %, de préférence de 20 à 70 % et encore de préférence de 30 à 60 % en poids de résine tackifiante, par rapport au poids total de la composition adhésive. Dans le cas où la composition selon l'invention comprend au moins une résine tackifiante, le rapport massique de composé polyvinyléther sur résine tackifiante peut être de 3 à 100 %.

Selon certains modes de réalisation, la composition adhésive comprend une seule résine tackifiante.

Selon des modes de réalisation alternatifs, la composition adhésive comprend des résines tackifiantes différentes, par exemple deux ou trois ou quatre ou cinq résines tackifiantes différentes.

Silsesquioxanes

Les silsesquioxanes sont typiquement des composés organiques de silicium pouvant adopter une structure polyhédrique ou une structure polymérique, avec des liaisons Si-O-Si. Ils ont typiquement la formule générale [Chem 26]

$[R'SiO_{3/2}]_t$

dans laquelle R', de nature identique ou différente, représente un radical organique, et t est un nombre entier pouvant varier de 6 à 12, de préférence t égale 6, 8, 10 ou 12.

Selon un mode de réalisation, le silsesquioxane a une structure polyhédrique (ou POSS pour « Polyhedral Oligomeric Silsesquioxane » en anglais).

De préférence, le silsesquioxane répond à la formule générale [Chem 27]

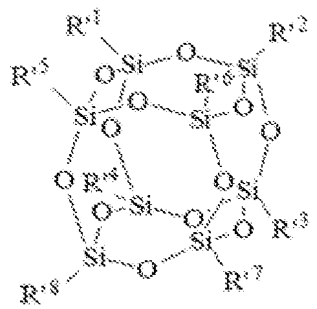

dans laquelle chacun de $R'^1$ à $R'^8$ représente, indépendamment les uns des autres, un groupe choisi parmi :

un atome d'hydrogène ;

un radical choisi parmi le groupe constitué d'un radical alcoxy linéaire ou ramifié en C1-C4, un radical alkyle linéaire ou ramifié comprenant de 1 à 30 atomes de carbone, un radical alcényle comprenant de 2 à 30 atomes de carbone, un radical aromatique comprenant de 6 à 30 atomes de carbone, un radical allyle comprenant de 3 à 30 atomes de carbone, un radical cyclique aliphatique comprenant de 3 à 30 atomes de carbone, un radical acyle comprenant de 1 à 30 atomes de carbone ; et

un groupe $-OSiR'^9R'^{10}$ dans lequel $R'^9$ et $R'^{10}$ représente chacun, indépendamment l'un de l'autre, un atome d'hydrogène ou un radical choisi dans le groupe constitué des alkyles linéaires ou ramifiés en C1-C4, des alcoxys linéaires ou ramifiés en C1-C4, des alcényles en C2-C4, d'un phényle, d'un radical allyle en C3-C6, d'un radical aliphatique cyclique en C3-C8, et d'un radical acyle en C1-C4 ;

à condition :

qu'au moins un radical parmi les radicaux $R'^1$ à $R'^8$ soit un radical alcoxy en C1-C4; et

qu'au moins un radical parmi les radicaux $R'^1$ à $R'^8$ soit un radical phényle. Les silsesquioxanes sont des composés connus qui sont décrits notamment dans la demande WO 2008/107331 A1. Certains sont également disponibles au plan commercial, ainsi le produit de DOW commercialisé sous la dénomination : DOW CORNING® 3074 et DOW CORNING® 3037 (numéro CAS = 68957-04-0).

La composition adhésive selon l'invention peut comprendre de 0 à 40 %, de préférence de 0 à 20 % et encore de préférence de 0 à 10 % en poids de silsesquioxane, par rapport au poids total de la composition adhésive

Charges

La charge peut être choisie parmi les charges organiques, les charges inorganiques et leurs mélanges.

A titre de charge(s) organique(s), on peut utiliser n'importe quelle(s) charge(s) organique(s) et notamment polymérique(s) typiquement utilisée(s) dans le domaine des adhésifs. On peut utiliser par exemple du polychlorure de vinyle (PVC), une(des) polyoléfine(s), du caoutchouc, de l'éthylène vinyl acétate (EVA), des fibres aramides telles que par exemple le KEVLAR ®, des microsphères creuses en polymère thermoplastique expansibles ou non expansibles (tel que par exemple des microsphères creuses en chlorure de vinylidène/acrylonitrile), un(des) polymère(s) thermoplastique(s) choisi(s) parmi ceux utilisés dans la préparation de HMPSA, tels que l'éthylène vinyl acétate (EVA), ou des copolymères blocs styréniques (tels que SIS, SBS, SIBS, SEBS, SEPS, et leurs dérivés greffés avec par exemple de l'anhydride maléïque).

La charge peut être un agent d'expansion (également appelé agent gonflant). La charge peut être sous forme de billes creuses, c'est-à-dire contenant un gaz, ou de billes susceptibles d'être expansées et former des billes creuses, c'est-à-dire contenant du vide ou un gaz.

De préférence, la charge est une charge inorganique.

Selon un mode de réalisation, la charge est choisie parmi le sable, la silice précipitée et/ou pyrogénée, les zéolithes, les billes de verre, le verre, le quartz, la barite, l'alumine, le mica, le talc, les carbonates de métaux alcalins ou alcalino-terreux (par exemple le carbonate de calcium).

La ou les charges représente(nt) de préférence de 0 % à 15 % en poids, de préférence de 0 % à 10 %, préférentiellement de 0 % à 5 % en poids, du poids total de la composition adhésive.

Selon un mode de réalisation, la composition selon l'invention ne comprend pas de charge.

Agent plastifiant

La composition selon l'invention peut comprendre au moins un agent plastifiant. La teneur totale en plastifiant(s) dans la composition peut aller de 0 % à 30 % en poids, de préférence de 1 % à 30 % en poids, voire par exemple de 1 % à 15 % en poids par rapport au poids total de ladite composition.

A titre d'exemple d'agent plastifiant utilisable, on peut utiliser n'importe quel agent plastifiant habituellement utilisé dans le domaine des adhésifs, tel que par exemples les phtalates, les benzoates, les esters de trimethylolpropane, les esters de triméthyloléthane, les esters de triméthylolméthane, les esters de glycérol, les esters de pentaerythritol, les huiles minérales napthéniques, les adipates, les cyclohexyldicarboxylates, les huiles paraffiniques, les huiles naturelles (éventuellement époxydées), les polypropylènes, les polybutylènes, les polyisoprènes hydrogénés, et leurs mélanges.

Parmi les phtalates, on peut par exemple citer le diisononyl phtalate, le di-isobutyl phtalate, le dioctyle phtalate, le dicyclohexyl phtalate, le diisooctyle phtalate, le diisododécyle phtalate, le dibenzyle phtalate ou le butylbenzyle phtalate.

Parmi les benzoates, on peut par exemple citer : le néopentylglycol dibenzoate (par exemple disponible sous la dénomination UNIPLEX® 512 auprès de LANXESS), le dipropylèneglycol dibenzoate (par exemple disponible sous la dénomination BENZOFLEX® 9-88SG auprès de EASTMAN), un mélange de diéthylène glycol dibenzoate et de dipropylène glycol dibenzoate (par exemple disponible sous la dénomination K-

FLEX® 850 S auprès de KALAMA CHEMICAL), ou encore un mélange de diéthylène glycol dibenzoate, de dipropylène glycol dibenzoate et de triéthylène glycol dibenzoate (par exemple disponible sous la dénomination BENZOFLEX® 2088 auprès de EASTMAN).

Parmi les esters de pentaérythritol, on peut par exemple citer le tétravalérate de pentaérythritol (par exemple disponible sous la dénomination PEVALEN™ auprès de la société PESTORP).

Parmi les cyclohexanedicarboxylates, on peut par exemple citer le diisononyl 1,2-cyclohexanedicarboxylate (par exemple disponible sous la dénomination HEXAMOLL DINCH® auprès de BASF).

Pigment

Lorsqu'un pigment est présent dans la composition selon l'invention, sa teneur est de préférence inférieure ou égale à 3 % en poids, de préférence encore inférieure ou égale à 2 % en poids, par rapport au poids total de la composition. Lorsqu'il est présent, le pigment peut par exemple représenter de 0,1 % à 3 % en poids ou de 0,4 % à 2 % en poids du poids total de la composition selon l'invention.

Les pigments peuvent être des pigments organiques ou inorganiques.

Par exemple, le pigment est $TiO_2$, en particulier le KRONOS® 2059 commercialisé par la société KRONOS.

Absorbeur d'humidité

L'absorbeur d'humidité, s'il est présent, peut être par exemple choisi parmi les dérivés alkoxysilane hydrolysables, non polymériques, de masse moléculaire inférieure à 500 g/mol, de préférence choisi parmi les dérivés de triméthoxysilane et de triéthoxysilane. Un tel agent peut typiquement prolonger la durée de conservation de la composition durant le stockage et le transport avant son utilisation. On peut par exemple citer le gamma- métacryloxypropyltriméthoxysilane (par exemple disponible sous la dénomination commerciale SILQUEST® A-174 auprès de la société MOMENTIVE), le méthacryloxyméthyltriméthoxysilane (par exemple disponible sous la dénomination GENIOSIL® XL33 auprès de WACKER), le vinyltriméthoxysilane, l'isooctyltriméthoxysilane, ou le phényltriméthoxysilane.

La teneur en absorbeur d'humidité est de préférence inférieure ou égale à 3 % en poids, de préférence encore inférieure ou égale à 2 % en poids par rapport au poids total de la composition selon l'invention. Lorsqu'il est présent, l'absorbeur d'humidité peut par exemple représenter de 0,1 % à 3 % en poids ou de 1 % à 2 % en poids par rapport au poids total de la composition.

La composition selon l'invention peut comprendre une quantité de 0,1 % à 3 %, de préférence de 1 % à 3 % en poids, d'au moins un stabilisant UV ou antioxydant. Ces composés sont typiquement introduits pour protéger la composition d'une dégradation résultant d'une réaction avec de l'oxygène qui est susceptible de se former par action de la chaleur ou de la lumière. Ces composés peuvent inclure des antioxydants primaires qui piègent les radicaux libres. Les antioxydants primaires peuvent être utilisés seuls ou en combinaison avec d'autres antioxydants secondaires ou des stabilisants UV. On peut par exemple citer l'IRGANOX® 1010, l'IRGANOX® B561, l'IRGANOX® 245, l'IRGAFOS® 168 commercialisés par BASF.

Composition mono-composante ou bi-composante

Selon certains modes de réalisation, la composition adhésive selon l'invention est une composition mono-composante. En d'autres termes une composition selon laquelle tous les composés sont conditionnés dans un même compartiment. Dans ce cas, la composition est de préférence prête à l'emploi c'est-à-dire que l'utilisateur (particulier ou professionnel) peut appliquer directement la composition adhésive sur le substrat à recouvrir et/ou le revêtement souple, sans avoir à effectuer de mélange préalable. Selon des modes de réalisation alternatifs, la composition adhésive selon l'invention est une composition bi-composante. En d'autres termes une composition selon laquelle les composants sont conditionnés dans deux compartiments distincts.

Dans ce cas, la composition bi-composante peut comprendre une partie A et une partie B, les deux parties étant mélangées avant l'utilisation et l'application de la composition adhésive sur une couche substrat par exemple. Les composants décrits ci-dessus peuvent être repartis dans la partie A et/ou dans la partie B de la composition bi-composante.

Selon des modes de réalisation préféré, la partie A de la composition bi-composante peut par exemple comprendre le polymère silylé (tel que décrit ci-dessus). La partie B de la composition peut dans ce cas comprendre le catalyseur de réticulation (tel que décrit ci-dessus). Le composé polyvinyléther ainsi que les additifs optionnels peuvent être présents dans la partie A et/ou la partie B de la composition. Les composants additionnels (autre additifs) peuvent être présents dans la partie A et/ou dans la partie B de la composition bi-composante.

Utilisation de la composition

La composition selon l'invention est utilisée pour fabriquer des articles auto-adhésifs. Par « article auto-adhésif » on entend tout article qui peut être collé sur une surface seulement par l'action d'une pression avec la main ou un équipement, sans l'utilisation de colles ou adhésifs supplémentaires.

Les articles auto-adhésifs peuvent comprendre une couche support revêtue d'une couche auto-adhésive, ladite couche auto-adhésive étant la composition adhésive selon l'invention à l'état réticulé.

De préférence, l'article auto-adhésif est un article auto adhésif sensible à la pression.

Ces articles ont notamment pour but d'être appliqués sur une surface à coller afin de rapprocher, de maintenir, de fixer, ou simplement d'immobiliser, d'exposer des formes, logos, images ou informations. Ces articles peuvent être utilisés dans de nombreux domaines, tels que le domaine médical, l'habillement, l'emballage, l'automobile (par exemple pour la pose de logos, le lettrage, l'insonorisation intérieure, l'habillage intérieur, les collages dans l'habitacle) ou la construction (par exemple pour l'isolation phonique et thermique, l'assemblage de fenêtres) ; en particulier dans le domaine médical et dans le domaine de la construction. Ils peuvent être façonnés en fonction de leur application finale, par exemple sous la forme de rubans, tels que les rubans à usage industriel, les rubans de bricolage ou à usage de fixation sur chantiers, les rubans simples ou double face, ou sous la forme d'étiquettes, de bandages, de pansements, de patchs ou de films graphiques.

Selon un mode de réalisation, l'article autoadhésif est un système multicouche autoadhésif, et en particulier une étiquette ou un ruban autoadhésif, pouvant être à simple ou double face.

Le matériau utilisable pour la couche support peut par exemple être tout type de support rigide ou souple. On peut par exemple citer les supports de type mousses, feutrines, support non tissés, plastiques, membranes, les papiers ou un film d'un matériau polymère à une ou plusieurs couches.

La couche support est en matériau par exemple choisi parmi les polyoléfines, telles que le polyéthylène, dont le polyéthylène haute densité, le polyéthylène faible densité, le polyéthylène faible densité linéaire et le polyéthylène ultra faible densité linéaire, le polypropylène et les polybutylènes ; le polystyrène ; le caoutchouc naturel ou synthétique ; les copolymères vinyliques, tels que le polychlorure de vinyle, plastifié ou non plastifié, et les poly(acétate de vinyle) ; les copolymères oléfiniques, tels que les copolymères éthylène/méthacrylate, les copolymères éthylène/acétate de vinyle, les copolymères acrylonitrile/butadiène/styrène, et les copolymères éthylène/propylène ; les polymères et les copolymères acryliques ; les polyuréthanes ; les polyéthers ; les polyesters ; et les mélanges de ceux-ci. De préférence, la couche support est à base de polymères acryliques, de polyéthylène (PE), polypropylène (PP) orienté, non orienté ou bi-orienté, polyimide, polyuréthane, polyester tel que le polyéthylène téréphtalate (PET), ou de papier.

Selon certains modes de réalisation, l'article autoadhésif obtenu à partir de la composition adhésive selon l'invention comprend une couche support permanente revêtue d'une couche adhésive. De préférence, la couche adhésive est en outre revêtue d'un film plastique ou papier protecteur antiadhérent, de préférence siliconé.

A titre d'alternative au film protecteur antiadhérent, la face arrière de la couche support permanente qui n'est pas revêtue de la couche adhésive, peut avoir une surface antiadhérente, par exemple une couche protectrice siliconée.

Selon un mode de réalisation, la couche support permanente est revêtue sur ses deux faces par une composition adhésive, qui peut être identique ou différente, au moins une des deux compositions adhésives étant selon l'invention.

De préférence, la couche support présente une épaisseur allant de 10 $\mu$m à 50 mm, de préférence encore allant de 10 $\mu$m à 20 mm, de préférence allant de 20 $\mu$m à 10 mm, de préférence encore allant de 20 $\mu$m à 1 mm.

Dans certains cas spécifiques, il est nécessaire de procéder à un traitement de surface de la couche support pour augmenter l'accroche de la couche adhésive lors de l'étape d'enduction sur celle-ci.

L'article autoadhésif selon l'invention peut ainsi coller deux substrats. Le substrat sur lequel l'article autoadhésif est destiné à être appliqué (désigné par « *substrat à coller* ») peut être flexible ou rigide. En particulier, il peut présenter les mêmes propriétés de flexibilité que la couche support décrite ci-dessus, de manière à être enroulé et conditionné sous la forme d'une bobine. Alternativement, le substrat à coller peut être rigide. Dans ce cas, le substrat ne peut être enroulé et conditionné sous la forme d'une bobine, par exemple telle que décrite précédemment. Le substrat à coller peut par exemple être choisi parmi le béton, le papier, les substrats de type polyoléfines ...

Selon certains modes de réalisation, l'article autoadhésif comprend en outre une couche antiadhérente protectrice (« *release liner »* en anglais).

Selon certains modes de réalisation, ladite couche antiadhérente est appliquée sur la couche adhésive, après réticulation de la composition adhésive.

La couche support peut être recouverte sur une de ses deux faces, la face arrière qui n'est pas revêtue de la couche adhésive, par une couche protectrice antiadhérente, par exemple par un film siliconé. De cette façon, l'article autoadhésif peut être enroulé sur lui-même puis déroulé sans problème grâce à l'absence d'adhésion de la couche adhésive sur la face siliconée.

L'article autoadhésif selon l'invention peut être susceptible d'être obtenu par le procédé comprenant les étapes suivantes :

- le mélange des parties A et B de la composition adhésive selon l'invention, dans le cas où il s'agit d'une composition bi-composante ;
- le chauffage de la composition adhésive à une température allant de 40°C à 130°C ;
- l'enduction par la composition adhésive d'une surface porteuse ;
- la réticulation de la composition adhésive enduite, par chauffage à une température allant de 50°C à 200°C en particulier dans un environnement gazeux où des molécules d'eau sont présentes entre 10 et 200 g par $m^3$ de gaz ;
- le contre collage ou le transfert de la couche adhésive réticulée sur une couche support ou sur un film protecteur anti-adhérent, ladite couche support ou film antiadhérent pouvant être le revers de la surface porteuse.

Par « surface porteuse » au sens de la présente invention, il faut comprendre soit un convoyeur à bande recouvert d'une couche antiadhérente, soit un film protecteur antiadhérent (« release liner » en anglais), soit une couche support. Ainsi, la surface porteuse est amenée à devenir partie intégrante de l'article auto-adhésif, soit comme film protecteur antiadhérent, soit comme couche support.

Dans le cas où la surface porteuse n'est pas une couche support, la dernière étape du procédé ci-dessus comprend le transfert de la couche adhésive réticulée sur une couche support.

Dans le cas où la surface porteuse est une couche support, la dernière étape du procédé ci-dessus comprend le contre collage de la couche adhésive sur un film protecteur antiadhérent.

Selon une variante préférée de l'invention, la dernière étape du procédé ci-dessus consiste en un transfert de la couche adhésive réticulée sur une couche support souple (pouvant être un film plastique) après refroidissement de la couche adhésive réticulée à une température inférieure à la température de dégradation ou de ramollissement du matériau composant la couche support.

Selon un mode de réalisation, l'article autoadhésif selon l'invention est susceptible d'être obtenu par le procédé tel que décrit précédemment, ne comprenant pas d'étape de prétraitement de la surface de la couche support. Ces prétraitements visent à modifier chimiquement et/ou physiquement ladite surface, pour accroitre l'énergie superficielle et/ou la rugosité de ladite surface, et ainsi améliorer l'adhérence de la couche adhésive sur ladite surface. A titre d'exemple, de traitements de surface connus, on peut citer un traitement plasma, corona, une abrasion ou encore l'application sur ladite surface d'un agent chimique d'accroche (dit aussi primaire) capable de conférer au substrat revêtu dudit agent une énergie de surface élevée.

Le procédé de fabrication de l'article autoadhésif selon l'invention peut comprendre en outre une étape d'enduction d'une seconde couche de composition adhésive selon l'invention sur la couche support suivie d'une étape de réticulation de la composition adhésive enduite par chauffage à une température allant de 20 à 200°C. Selon ce mode de réalisation, un article auto-adhésif double face est obtenu.

Le ou les étapes d'enduction peuvent être réalisée au moyen de dispositifs d'enduction connus, comme par exemple une buse à lèvre ou de type rideau, ou encore au rouleau. Ces étapes peuvent mettre en œuvre un grammage de composition adhésive allant de 3 à 5 000 g/$m^2$. Le grammage de composition adhésive nécessaire pour la fabrication d'étiquettes autoadhésives peut aller de 10 à 100 g/$m^2$, et de préférence de 20 à 50 g/$m^2$. Celui nécessaire pour la fabrication de rubans auto-adhésifs peut varier dans un domaine beaucoup plus large allant de 3 à 5 000 g/$m^2$, et de préférence de 15 à 250 g/$m^2$ par face.

Selon certains modes de réalisation, la composition adhésive enduite est en outre soumise, lors de l'étape de réticulation à un traitement dans une atmosphère humide caractérisée par son niveau d'humidité. De préférence, l'atmosphère humide est une atmosphère dans laquelle de 2 à 100 % des molécules sont des molécules d'eau, de préférence de 3 à 50 %, de préférence encore de 3 à 10 % des molécules sont des molécules d'eau.

Le taux d'humidité est exprimé en pourcentage d'eau par unité de volume, ce qui correspond au nombre de molécules d'eau divisé par le nombre total de molécules dans une unité de volume. Grâce à la nature linéaire de cette échelle, le taux d'humidité est facilement mesuré et contrôlé en utilisant par exemple des moniteurs de type P.I.D (« Proportional-Integral-Derivative »). Le pourcentage en poids peut être calculé en multipliant le pourcentage du nombre de molécules d'eau par rapport au nombre total de molécules par un facteur de 0,622. Des informations générales sur le taux d'humidité dans divers environnements sont décrites par W. Wagner et al., dans « International Steam Tables - Properties of Water and Steam based on the Industrial Formulation IAPWS-IF97 ».

L'étape de réticulation thermique a notamment pour effet la création - entre les chaînes polymériques à terminaisons alcoxysilanes hydrolysables de la composition adhésive et sous l'action de l'humidité atmosphérique - des liaisons de type siloxane qui conduisent à la formation d'un réseau polymérique tri-dimensionnel. La composition adhésive ainsi réticulée est en particulier un adhésif sensible à la pression qui confère à la couche support qui en est revêtue le pouvoir adhésif et le tack désirables.

De préférence, l'enduction est réalisée uniformément sur la couche support ou sur la couche protectrice anti-adhérente mais l'enduction peut également être adaptée à la forme souhaitée de l'article auto-adhésif final.

Selon un mode de réalisation, l'enduction par la composition adhésive est réalisée sur au moins une partie des deux faces de la couche support. Si les deux faces de la couche support sont enduites, la composition adhésive peut être

identique ou différente sur les deux faces, et le grammage peut être identique ou différent sur les deux faces.

Selon certains modes de réalisation de l'invention, l'article auto-adhésif comprend une couche adhésive sur au moins une partie d'une face ou sur au moins une partie des deux faces de la couche support, ladite ou lesdites couches adhésives étant éventuellement revêtues d'une couche protectrice anti-adhérente. Selon un mode de réalisation, l'article auto-adhésif comprend deux couches protectrices anti-adhérentes sur chacune des deux couches adhésives. Dans ce cas, les deux couches protectrices peuvent être en matériaux identiques ou différents et/ou elles peuvent avoir une épaisseur identique ou différente.

Les articles autoadhésifs selon la présente invention sont des articles imper-respirants. Par « *imper-respirant »,* on entend perméable à la vapeur d'eau et imperméable à l'eau liquide.

De préférence, l'article obtenu à partir de la composition selon l'invention présente une perméabilité à la vapeur d'eau (MVTR, pour « Moisture Vapour Transmission Rate ») d'au moins 350 g/$m^2$ par 24 heures, à 37°C, à un taux d'humidité relative de 50 %, pour une épaisseur de film de 30 $\mu$m. De manière plus préférée, la perméabilité à la vapeur d'eau MVTR du film vaut au moins 500 g/$m^2$/24 h, de préférence au moins 600 g/$m^2$/24 h, de préférence encore au moins 700 g/$m^2$/24 h, et encore de préférence de 800 g/$m^2$/24 h, à 37°C, à un taux d'humidité relative de 50 %, pour une épaisseur de film de 30 $\mu$m. En particulier, la perméabilité MVTR de la membrane peut valoir de 350 à 400 g/$m^2$/24 h, ou de 400 à 500 g/$m^2$/24 h, ou de 500 à 600 g/$m^2$/24 h, ou de 600 à 700 g/$m^2$/24 h, ou de 700 à 800 g/$m^2$/24 h, ou de 800 à 900 g/$m^2$/24 h, ou de 900 à 1000 g/$m^2$/24 h, ou de 1000 à 1200 g/$m^2$/24 h, ou 1200 à 1500 g/$m^2$/24 h, ou de 1500 à 2000 g/$m^2$/24 h, ou de 2000 à 2500 g/$m^2$/24 h, ou de 2500 à 3000 g/$m^2$/24 h, ou de 3000 à 3500 g/$m^2$/24 h, ou de 3500 à 4000 g/$m^2$/24 h, ou de 4000 à 4500 g/$m^2$/24 h, ou de 4500 à 5000 g/$m^2$/24 h, à 37°C, à un taux d'humidité relative de 50 %, pour une épaisseur de film de 30 $\mu$m. La perméabilité à la vapeur d'eau (MVTR) du film, à 37°C, pour un taux d'humidité relative de 50 %, pour une épaisseur de film de 30 $\mu$m, peut être mesurée selon la norme ASTM E96 B. L'article autoadhésif selon l'invention peut être utilisé dans une méthode de collage comprenant les étapes suivantes :

- retirer la couche protectrice anti-adhérente, lorsqu'une telle couche est présente ;
- appliquer l'article auto-adhésif sur une surface d'un produit; et
- appliquer une pression sur ledit article.

A la deuxième étape, l'article auto-adhésif est appliqué de manière à ce que la partie autoadhésive de l'article (formée par la couche auto-adhésive) soit face à la surface du produit.

Selon certains modes de réalisation dans lequel l'article auto-adhésif est un article double face, la méthode de collage comprend en outre une étape dans laquelle soit une seconde surface d'un produit est appliquée sur l'article collé sur la première surface d'un produit, soit l'article collé sur la première surface d'un produit est appliqué sur une seconde surface d'un produit.

## Exemples

Les exemples suivants illustrent l'invention sans la limiter.

<u>Performance adhésion « PSA »</u>

Les compositions adhésives ont été appliquées sur une couche substrat de polytéréphtalate d'éthylène (PET) de sorte à former une couche d'une épaisseur de 50 $\mu$m.

Les couches substrats revêtues des compositions adhésives ont été stockées pendant 7 jours à 70°C afin d'assurer la réticulation complète de la composition.

La performance d'adhésion des compositions a été mesurée par un test de pelage à 180° selon la norme Finat 1. Ainsi, selon cette norme, les 2/3 d'une éprouvette d'adhésif (15cm x 2,5 cm) est positionnée sur une plaque de test (inox poncé, HDPE etc). Un rouleau de 2 kg est passé deux fois sur l'éprouvette pour promouvoir la mouillabilité. Un temps d'attente de 10 minutes est respecté avant la traction. L'éprouvette est positionnée en parallèle de la traction, et est repliée sur elle-même selon un angle de 180°C. La partie de l'éprouvette non collée sur la plaque de test permet d'avoir une prise pour la mâchoire et ainsi permettre la traction selon une vitesse normée de 300 mm/min. L'appareil mesure la force requise au décollement de l'éprouvette selon les conditions décrites précédemment.

Le principe de ce test consiste en la détermination de la force nécessaire à la séparation (ou pelage) de la couche de la composition adhésive de la couche substrat.

Le pouvoir collant (tack) des compositions a été mesuré selon la norme Finat 9. Ainsi, selon cette norme, une éprouvette d'adhésif de 15 cm sur 2,5 cm est repliée en forme de boucle, l'adhésif étant la face extérieure de la boucle. A la vitesse de 300 mm/min, la boucle est rapprochée puis déposée délicatement sur une plaque (verre). Une fois un carré de 1 $inch^2$ déposé sur la plaque, la boucle est retirée de la plaque et la force nécessaire au retrait de l'adhésif est mesurée.

Perméabilité à la vapeur d'eau (MVTR)

Ce test permet de mesurer la vapeur d'eau pouvant traverser la couche de la composition adhésive.

Ce test a été effectué en appliquant la norme NF EN 13726-2 à 37°C

Les compositions adhésives ont été appliquées sur une couche substrat de matériau non-tissé (NW) ayant une MVTR de 5 861 g/$m^2$/24h ou sur une couche substrat de polyuréthane (PU) ayant un MVTR de 7 826 g/$m^2$/24h, de sorte à former une couche d'une épaisseur de 0.03 mm sans le backing (support pour renforcer le film PU lors de la lamination, épaisseur de 0.104 mm avec backing).

Les couches substrats revêtues des compositions adhésives ont été stockées pendant 7 jours à 70°C afin d'assurer la réticulation complète de la composition.

Exemple 1 :

Les composants suivants ont été utilisés pour préparer une composition A (selon l'invention) et une composition B (référence) :
- CLEARTACK® W100, disponible auprès de la société CRAY VALLEY, est une résine tackifiante obtenue par polymérisation d'alpha-méthyl styrène sans action de phénols, ayant une masse moléculaire moyenne en nombre de 900 Da ;
- SYLVALITE® RE 100, disponible auprès de la société ARIZONA CHEMICAL, est une résine de type colophane, ayant masse moléculaire moyenne en nombre est d'environ 1700 Da ;
- Lutonal® M40, disponible auprès de BASF, est un polyvinyléther;
- IRGANOX® 1010, disponible auprès de BASF, est un antioxydant de type phénol encombré ;
- SPUR Y-19204, disponible auprès de la société MOMENTIVE, est un prépolymère polyuréthane polypropylène glycol silylé ayant des terminaisons de triméthoxysilane et ayant un poids moléculaire moyen Mn de 24 000 g/mol ;
- K-KAT® 5218, disponible auprès de la société KING INDUSTRIES, est un catalyseur de type chélate d'aluminium.

[Tableau 1]

| Compositions (%) | A (inv) | B (ref) |
|---|---|---|
| CLEARTACK ® W100 | 18,48 | 23,43 |
| SYLVALITE ® RE 100 | 18,48 | 23,43 |
| Lutonal ® M40 | 9,90 | - |
| IRGANOX ® 1010 | 0,50 | 0,50 |
| SPUR Y-19204 | 51,64 | 51,64 |
| K-KAT ® 5218 | 1,00 | 1,00 |

La composition B est dépourvue de composé polyvinyléther.

La performance à l'adhésion et la perméabilité à la vapeur ont été mesurées comme détaillé ci-dessus. Les résultats sont illustrés dans le tableau ci-dessous :

[Tableau 2]

| Performances à 50 gsm Compositions | A (inv) | B (réf) |
|---|---|---|
| 180° pelage SS (N/inch)/PET | 15,2 +/- 0,51 AF | 17,2 +/- 0,13 AF |
| 180° peel HDPE (N/inch)/PET | 10,3 +/- 0,74 AF | 15,4 +/- 0,35 AF |
| Pouvoir « tack » (N/inch)/PET | 19,2 +/-1,4 AF | 18,5 +/- 2,4 AF |
| MVTR (g/$m^2$/24h)/NW | 1107 +/- 51 | 865 +/- 60 |

Légende :

- SS = inox poncé
- AF = « Adhesive Failure » (pas de résidu qui reste, la perte d'adhésion explique le résultat du test, le produit a une bonne cohésion)

- HDPE= « High density polyethylene » (polyéthylène de haute densité)

On constate que la composition A selon l'invention permet d'obtenir des articles ayant une perméabilité à la vapeur améliorée, sans compromettre pour autant les bonnes propriétés d'adhésion.

Exemple 2

Les composants suivants ont été utilisés pour préparer une composition C (selon l'invention) et une composition D (référence) :
- PICCO® AR100, disponible auprès de la société EASTMAN, est une résine d'hydrocarbures;
- Lutonal® M40, disponible auprès de BASF, est un polyvinyléther;
- GENIOSIL® STPE-30, disponible auprès de la société WACKER, est un polyéther ayant des terminaisons diméthoxy(méthyl)silylméthylcarbamate et ayant un poids moléculaire moyen de 24 000 g/mol;
- K-KAT® 5218, disponible auprès de la société KING INDUSTRIES, est un catalyseur de type chélate d'aluminium.

[Tableau 3]

| Compositions (%) | C (inv) | D (réf) |
|---|---|---|
| PICCO ® AR100 | 44,14 | 53,36 |
| Lutonal ® M40 | 10,22 | - |
| GENIOSIL ® STPE-30 | 44,64 | 44,64 |
| K-KAT ® 5218 | 1,00 | 1,00 |

La composition D est dépourvue de composé polyvinyléther.

La performance à l'adhésion et la perméabilité à la vapeur ont été mesurées comme détaillé ci-dessus. Les résultats sont illustrés dans le tableau [tableau 4] ci-dessous :

[Tableau 4]

| Compositions Performances à 50 gsm | C (inv) | D (réf) |
|---|---|---|
| 180° pelage SS (N/inch)/PET | 32,5 +/- 0,85 AF | 34,2 +/- 2,5 AF |
| 180° peel HDPE high density polyethylene, (N/inch)/PET | 22,2 +/- 0,70 AF | 24,7 +/- 0.34 AF |
| Pouvoir « tack » (N/inch)/PET | 39,7 +/-2,9 AF | 48,4 +/- 1,0 AF |
| MVTR ($g/m^2/24h$)/NW | 772 +/- 32 | 488 +/-118 |

On constate que la composition C selon l'invention permet d'obtenir des articles ayant une perméabilité à la vapeur améliorée, sans compromettre pour autant les bonnes propriétés d'adhésion.

Exemple 3 :

Les composants suivants ont été utilisés pour préparer une composition E (selon l'invention) et une composition F (comparative) :

- CLEARTACK® W100, disponible auprès de la société CRAY VALLEY, est une résine tackifiante obtenue par polymérisation d'alpha-méthyl styrène sans action de phénols, ayant une masse moléculaire moyenne en nombre de 900 Da ;
- SYLVALITE® RE 100, disponible auprès de la société ARIZONA CHEMICAL, est une résine de type colophane, ayant masse moléculaire moyenne en nombre est d'environ 1 700 Da ;
- Lutonal® M40, disponible auprès de BASF, est un polyvinyléther ;
- IRGANOX® 1010, disponible auprès de BASF, est un antioxydant de type phénol encombré ;
- SPUR Y-19204, disponible auprès de la société MOMENTIVE,est un prépolymère polyuréthane polypropylène glycol silylé ayant des terminaisons de triméthoxysilane et ayant un poids moléculaire moyen Mn de 24 000g/mol
- TnBT est un catalyseur de type tétrabutanolate de titane.

[Tableau 5]

| Compositions (%) | E (inv) | F (comp) |
|---|---|---|
| CLEARTACK® W100 | 13,5 | 23,4 |
| SYLVALITE® RE 100 | 13,5 | 23,4 |
| Lutonal® M40 | 19,8 | - |
| IRGANOX® 1010 | 0,5 | 0,5 |
| SPUR Y-19204 | 51,6 | 51,6 |
| TnBT | 1 | 1 |

La composition F est dépourvue de composé polyvinyléther.

La performance à l'adhésion et la perméabilité à la vapeur ont été mesurées comme détaillé ci-dessus. Les résultats sont illustrés dans le tableau ci-dessous :

[Tableau 6]

| Compositions 150 gsm | E (inv) | F (comp) |
|---|---|---|
| 180° pelage SS (N/inch)/PET | 16,9 +/- 0,1 AF | 18,1 +/- 0,4 AF |
| MVTR (g/m$^2$/24h)/PU | 395,6 +/- 6,04 | 215,6 +/- 2,77 |

On constate que la composition E selon l'invention permet d'obtenir des articles ayant une perméabilité à la vapeur améliorée, sans compromettre pour autant les bonnes propriétés d'adhésion.

## Revendications

**1.** Composition adhésive comprenant :

au moins un polymère silylé comprenant au moins un groupement alkoxisilane hydrolysable ;
au moins un composé polyvinyléther ; et
au moins un catalyseur de réticulation.,
dans laquelle le composé polyvinyléther est un homopolymère choisi parmi le poly(méthylvinyléther), le poly(éthylvinyléther), le poly(butylvinyléther), le poly(isobutylvinyléther), le poly(isopropylvinyléther), le poly(propylvinyléther), le poly(octylvinyléther), et leurs mélanges.

**2.** Composition selon la revendication 1 dans laquelle le polymère silylé est choisi parmi :

un polymère silylé de formule générale [Chem 2]

$$(R^5O)_{3-p}(R^4)_pSi- R^0- O-R^2-O-R^0- Si(R^4)p(OR^3)_{3-p}$$

un polymère silylé de formule générale [Chem 3]

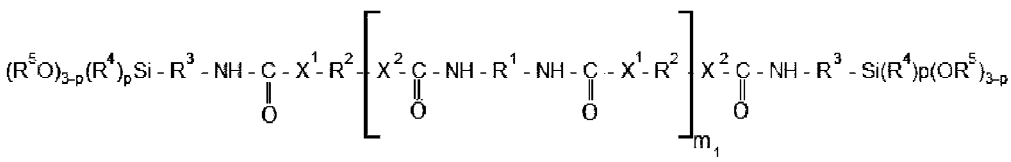

un polymère silylé de formule générale [Chem 4]

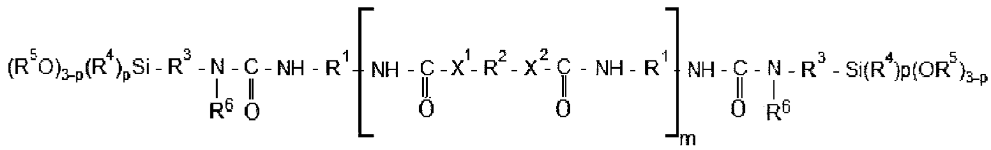

un polymère silylé de formule générale [Chem 5]

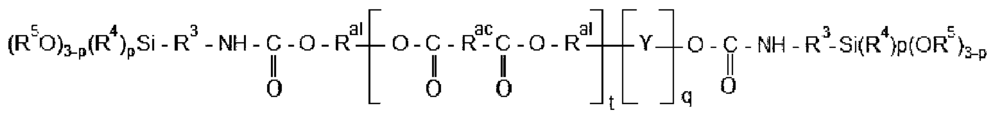

dans lesquelles :

$X^1$ et $X^2$ représentent, indépendamment les uns des autres, un atome d'oxygène ou un groupement -NH- ;
$R^1$ représente un radical divalent hydrocarboné comprenant de 5 à 15 atomes de carbone qui peut être aromatique ou aliphatique, linéaire, ramifié ou cyclique ;
$R^0$ représente un radical divalent alkylène linéaire ou ramifié comprenant de 3 à 6 atomes de carbone ;
$R^3$ représente un radical divalent alkylène linéaire ou ramifié comprenant de 1 à 6 atomes de carbone, de préférence $R^3$ représentant méthylène ou n-propylène ;
$R^2$ représente un bloc polyéther $-R^{pe}-[OR^{pe}]_n-$ dans lequel $R^{pe}$ représente un radical divalent alkylène linéaire ou ramifié comprenant de 2 à 4 atomes de carbone ;
$R^4$ et $R^5$, identiques ou différents, représentent chacun un radical alkyl linéaire ou ramifié comprenant de 1 à 4 atomes de carbone ;
$R^6$ représente un atome d'hydrogène, un radical phényle, un radical alkyle linéaire, ramifié ou cyclique comprenant de 1 à 6 atomes de carbone, ou un radical 2-succinate de formule générale [Chem 6]

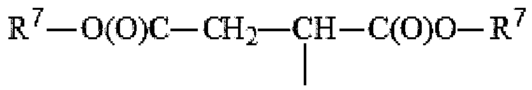

dans laquelle $R^7$ est un radical alkyle linéaire ou ramifié comprenant de 1 à 6 atomes de carbone ;
n est un nombre entier tel que la masse moléculaire moyenne en nombre du bloc polyéther $-[OR^{pe}]_n-$ va de 300 g/mol à 40 000 g/mol dans les polymères de formules générales [Chem 2], [Chem 3] et [Chem 4] ;
$m_1$ est zéro ou un nombre entier ;
$m_1$ sont tel que la masse moléculaire moyenne en nombre du polymère de formule générale [Chem 3] va de 500 g/mol à 50 000 g/mol, de préférence de 700 g/mol à 20 000 g/mol ;
m est un nombre entier différent de zéro ;
m est tel que la masse moléculaire moyenne en nombre du polymère de formule générale [Chem 4] va de 500 g/mol à 50 000 g/mol, de préférence de 700 g/mol à 20 000 g/mol ;
p est un nombre entier égal à 0, 1 ou 2, p étant de préférence 0 ou 1 ;
$R^{al}$ représente un radical hydrocarboné divalent issu d'un diol par remplacement de chacun des deux groupes hydroxyles par une valence libre ou représente le radical $R^2$;
$R^{ac}$ représente un radical hydrocarboné divalent issu d'un acide dicarboxylique par remplacement de chacun des deux groupes carboxyles COOH par une valence libre ;
$[Y]_q$ représente un motif de répétition de formule générale [Chem 28]

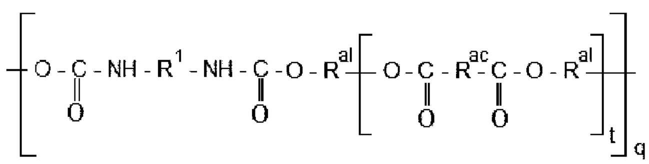

t est un nombre tel que le polyester diol de formule générale [Chem 7]

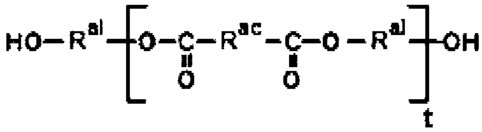

a un indice d'hydroxyle IOH compris entre 4 et 60 mg KOH/g ;
q est un nombre entier différent de zéro ;
t et q sont tels que la masse moléculaire moyenne en nombre du polymère de formule générale [Chem 5] est comprise entre 400 g/mol et 50 000 g/mol, ledit indice hydroxyle IOH et ladite masse moléculaire moyenne étant déterminés selon la norme ISO 14900:2001.

**3.** Composition selon l'une des revendications précédentes, dans laquelle le catalyseur de réticulation est choisi dans le groupe constitué des amines, des composés organo-métalliques, des acides et de leurs dérivés, et de leurs mélanges.

**4.** Composition selon l'une des revendications précédentes, comprenant en outre une résine tackifiante ; de préférence une résine tackifiante choisie parmi les résines terpènes-phénoliques, les résines d'hydrocarbures, les résines de colophane ; les résines acryliques et leurs mélanges.

**5.** Composition selon l'une des revendications précédentes, dans laquelle le composé polyvinyléther présente une valeur K de 40 à 120, et de préférence de 40 à 70, ladite valeur K étant mesurée selon la norme ISO 1628-1.

**6.** Composition selon l'une quelconque des revendications précédentes, dans laquelle le composé polyvinyléther présente une température de transition vitreuse de -60 à 0°C, et de préférence de -50 à -5°C, ladite température de transition vitreuse étant mesurée selon la norme NF EN ISO 11357-2.

**7.** Composition selon l'une des revendications précédentes, dans laquelle le composé polyvinyléther a une teneur de 1 à 60 % en poids, de préférence de 5 à 40 % en poids et de préférence de 5 à 25 % en poids par rapport au poids total de la composition.

**8.** Composition selon l'une des revendications précédentes, comprenant en outre au moins un silsesquioxane.

**9.** Composition selon l'une des revendications précédentes, ladite composition étant une composition mono-composante.

**10.** Composition l'une quelconque des revendications 1 à 9, ladite composition étant une composition bi-composante comprenant :

une partie A comprenant ledit au moins un polymère silylé ; et
une partie B comprenant ledit au moins un catalyseur de réticulation ledit au moins un composé polyvinyléther étant présent dans la partie A et/ou dans la partie B de la composition.

**11.** Utilisation de la composition selon l'une des revendications précédentes comme adhésif.

**12.** Article auto-adhésif comprenant au moins une couche support et au moins une couche de la composition selon l'une des revendications précédentes.

**13.** Article autoadhésif selon la revendication 12, ledit article étant choisi parmi les pansements, les bandages et les rubans médicaux.

## Patentansprüche

**1.** Klebstoffzusammensetzung, die Folgendes umfasst:

mindestens ein Silylpolymer, das mindestens eine hydrolysierbare Alkoxysilangruppe umfasst;
mindestens eine Polyvinylether-Verbindung und
mindestens einen Vernetzungskatalysator;

wobei es sich bei der Polyvinylether-Verbindung um ein Homopolymer handelt, das aus einem Poly(methylvinylether), Poly(ethylvinylether), Poly(butylvinylether), Poly(isobutylvinylether), Poly(isopropylvinylether), Poly(propylvinylether), Poly(octylvinylether) und deren Mischungen ausgewählt ist.

**2.** Zusammensetzung nach Anspruch 1, wobei das Silylpolymer ausgewählt ist aus:

einem Silylpolymer der allgemeinen Formel [Chem 2],

$$(R^5O)_{3-p}(R^4)_pSi\text{-}R^0\text{-}O\text{-}R^2\text{-}O\text{-}\ R^0\text{-}Si(R^4)p(OR^5)_{3-p}$$

einem Silylpolymer der allgemeinen Formel [Chem 3],

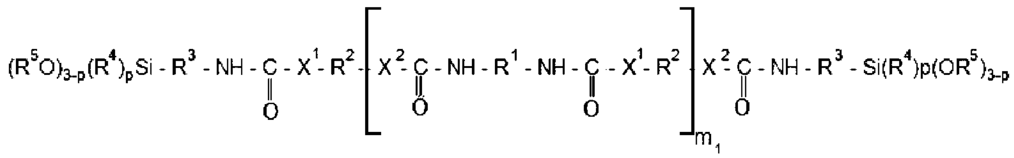

einem Silylpolymer der allgemeinen Formel [Chem 4],

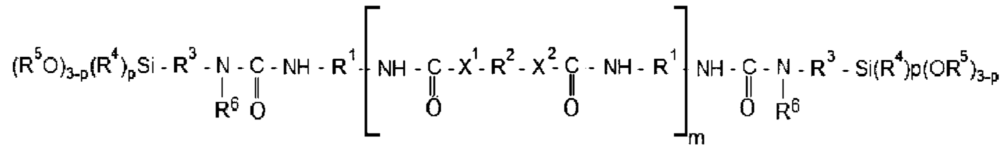

einem Silylpolymer der allgemeinen Formel [Chem 5],

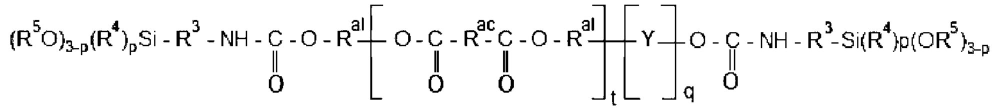

wobei:

$X^1$ und $X^2$ unabhängig voneinander für ein Sauerstoffatom oder eine -NH--Gruppe stehen;
$R^1$ für einen 5 bis 15 Kohlenstoffatome umfassenden zweiwertigen Kohlenwasserstoffrest steht, der aromatisch oder aliphatisch, geradkettig, verzweigt oder cyclisch sein kann;
$R^0$ für einen 3 bis 6 Kohlenstoffatome umfassenden geradkettigen oder verzweigten zweiwertigen Alkylenrest steht;
$R^3$ für einen 1 bis 6 Kohlenstoffatome umfassenden geradkettigen oder verzweigten zweiwertigen Alkylenrest steht, wobei $R^3$ vorzugsweise für Methylen oder n-Propylen steht;
$R^2$ für einen Polyetherblock $-R^{pe}-[OR^{pe}]_n-$ steht, wobei $R^{pe}$ für einen 2 bis 4 Kohlenstoffatome umfassenden geradkettigen oder verzweigten zweiwertigen Alkylenrest steht;
$R^4$ und $R^5$ gleich oder verschieden sind und jeweils für einen 1 bis 4 Kohlenstoffatome umfassenden geradkettigen oder verzweigten Alkylrest stehen;
$R^6$ für ein Wasserstoffatom, einen Phenylrest, einen 1 bis 6 Kohlenstoffatome umfassenden geradkettigen, verzweigten oder cyclischen Alkylrest oder einen 2-Succinat-Rest der allgemeinen Formel [Chem 6],

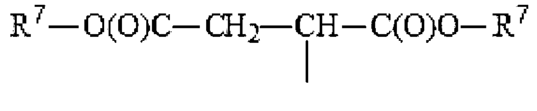

steht, wobei $R^7$ ein 1 bis 6 Kohlenstoffatome umfassender geradkettiger oder verzweigter Alkylrest ist;
n eine solche ganze Zahl ist, dass das Zahlenmittel der Molmasse des Polyetherblocks $-[OR^{pe}]_n-$ in den Polymeren der allgemeinen Formeln [Chem 2], [Chem 3] und [Chem 4] 300 g/mol bis 40 000 g/mol beträgt;
$m_1$ null oder eine ganze Zahl ist;
$m_1$ derart ist, dass das Zahlenmittel der Molmasse des Polymers der allgemeinen Formel [Chem 3] 500 g/mol

bis 50 000 g/mol, vorzugsweise 700 g/mol bis 20 000 g/mol, beträgt;
m eine von null verschiedene ganze Zahl ist;
m derart ist, dass das Zahlenmittel der Molmasse des Polymers der allgemeinen Formel [Chem 4] 500 g/mol bis 50 000 g/mol, vorzugsweise 700 g/mol bis 20 000 g/mol, beträgt;
p eine ganze Zahl gleich 0, 1 oder 2 ist, wobei p vorzugsweise 0 oder 1 ist;
$R^{al}$ für einen zweiwertigen Kohlenwasserstoffrest steht, der von einem Diol stammt, in dem jede der zwei Hydroxylgruppen durch eine freie Valenz ersetzt ist, oder für den Rest $R^2$ steht;
$R^{ac}$ für einen zweiwertigen Kohlenwasserstoffrest steht, der von einer Dicarbonsäure stammt, in der jede der zwei Carboxylgruppen COOH durch eine freie Valenz ersetzt ist;
$[Y]_q$ für eine Repetiereinheit der allgemeinen Formel [Chem 28] steht

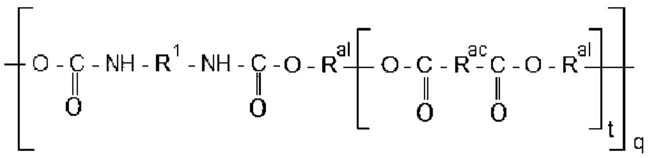

t eine solche Zahl ist, dass das Polyesterdiol der allgemeinen Formel [Chem 7]

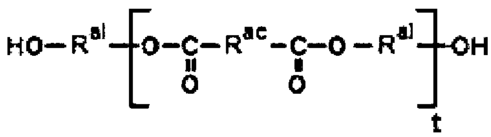

eine Hydroxylzahl IOH zwischen 4 und 60 mg KOH/g aufweist;
q eine von null verschiedene ganze Zahl ist;
t und q derart sind, dass das Zahlenmittel der Molmasse des Polymers der allgemeinen Formel [Chem 5] zwischen 400 g/mol und 50 000 g/mol beträgt, wobei die Hydroxylzahl IOH und die mittlere Molmasse gemäß der Norm ISO 14900:2001 bestimmt werden.

3. Zusammensetzung nach einem der vorhergehenden Ansprüche, wobei der Vernetzungskatalysator ausgewählt ist aus der Gruppe bestehend aus Aminen, organometallischen Verbindungen, Säuren und Derivaten davon und Mischungen davon.

4. Zusammensetzung nach einem der vorhergehenden Ansprüche, die außerdem ein klebrigmachendes Harz; vorzugsweise ein aus Terpin-Phenol-Harzen, Kohlenwasserstoffharzen, Kolophoniumharzen, Acrylharzen und Mischungen davon ausgewähltes klebrigmachendes Harz, umfasst.

5. Zusammensetzung nach einem der vorhergehenden Ansprüche, wobei die Polyvinylether-Verbindung einen K-Wert von 40 bis 120 und vorzugsweise von 40 bis 70 aufweist, wobei der K-Wert gemäß der Norm ISO 1628-1 gemessen wird.

6. Zusammensetzung nach einem der vorhergehenden Ansprüche, wobei die Polyvinylether-Verbindung eine Glasübergangstemperatur von -60 bis 0 °C und vorzugsweise von -50 bis -5 °C aufweist, wobei die Glasübergangstemperatur gemäß der Norm NF EN ISO 11357-2 gemessen wird.

7. Zusammensetzung nach einem der vorhergehenden Ansprüche, wobei die Polyvinylether-Verbindung einen Gehalt von 1 bis 60 Gew.-%, vorzugsweise 5 bis 40 Gew.-% und vorzugsweise 5 bis 25 Gew.-%, bezogen auf das Gesamtgewicht der Zusammensetzung, aufweist.

8. Zusammensetzung nach einem der vorhergehenden Ansprüche, die weiterhin mindestens ein Silsesquioxan umfasst.

9. Zusammensetzung nach einem der vorhergehenden Ansprüche, wobei es sich bei der Zusammensetzung um eine Einkomponenten-Zusammensetzung handelt.

10. Zusammensetzung nach einem der Ansprüche 1 bis 9, wobei es sich bei der Zusammensetzung um eine Zweikomponenten-Zusammensetzung handelt, die Folgendes umfasst:

einen Teil A, der das mindestens eine Silylpolymer umfasst; und
einen Teil B, der den mindestens einen Vernetzungskatalysator umfasst, wobei die mindestens eine Polyvinylether-Verbindung in Teil A und/oder in Teil B der Zusammensetzung vorhanden ist.

**11.** Verwendung der Zusammensetzung nach einem der vorhergehenden Ansprüche als Klebstoff.

**12.** Selbstklebender Artikel, der mindestens eine Trägerschicht und mindestens eine Schicht der Zusammensetzung nach einem der vorhergehenden Ansprüche umfasst.

**13.** Selbstklebender Artikel nach Anspruch 12, wobei der Artikel aus Pflastern, Verbänden und medizinischen Bändern ausgewählt ist.

## Claims

**1.** Adhesive composition comprising:

at least one silyl polymer comprising at least one hydrolyzable alkoxysilane group;
at least one polyvinyl ether compound; and
at least one curing catalyst,
in which the polyvinyl ether compound is a homopolymer chosen from poly(methyl vinyl ether), poly(ethyl vinyl ether), poly(butyl vinyl ether), poly(isobutyl vinyl ether), poly(isopropyl vinyl ether), poly(propyl vinyl ether), poly(octyl vinyl ether), and mixtures thereof.

**2.** Composition according to Claim 1, in which the silyl polymer is chosen from:

a silyl polymer of general formula [Chem 2]

$$(R^5O)_{3-p}(R^4)_pSi\text{-}R^0\text{-}O\text{-}R^2\text{-}O\text{-}R^0\text{-}Si(R^4)p(OR^5)_{3-p}$$

a silyl polymer of general formula [Chem 3]

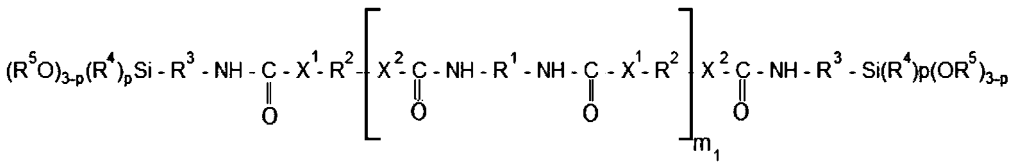

a silyl polymer of general formula [Chem 4]

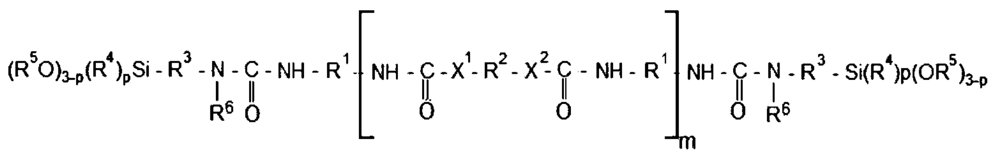

a silyl polymer of general formula [Chem 5]

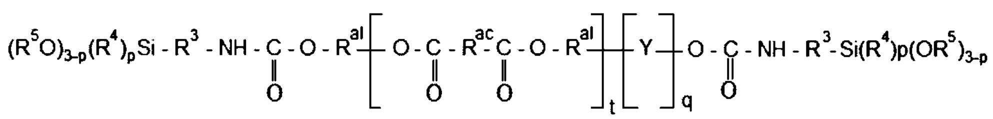

in which:

$X^1$ and $X^2$ represent, independently of each other, an oxygen atom or an -NH- group;

$R^1$ represents a divalent hydrocarbon-based radical comprising from 5 to 15 carbon atoms, which may be aromatic or aliphatic, linear, branched or cyclic;
$R^0$ represents a linear or branched divalent alkylene radical comprising from 3 to 6 carbon atoms;
$R^3$ represents a linear or branched divalent alkylene radical comprising from 1 to 6 carbon atoms, $R^3$ preferably representing methylene or n-propylene;
$R^2$ represents a polyether block $-R^{pe}-[OR^{pe}]_n-$ in which $R^{pe}$ represents a linear or branched divalent alkylene radical comprising from 2 to 4 carbon atoms;
$R^4$ and $R^5$, which may be identical or different, each represent a linear or branched alkyl radical comprising from 1 to 4 carbon atoms;
$R^6$ represents a hydrogen atom, a phenyl radical, a linear, branched or cyclic alkyl radical comprising from 1 to 6 carbon atoms, or a 2-succinate radical of formula [Chem 6]

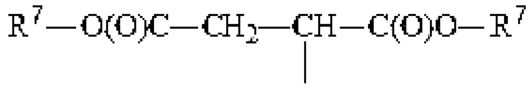

in which $R^7$ is a linear or branched alkyl radical comprising from 1 to 6 carbon atoms;
n is an integer such that the number-average molecular mass of the polyether block $-[OR^{pe}]_n-$ ranges from 300 g/mol to 40 000 g/mol in the polymers of general formulae [Chem 2], [Chem 3] and [Chem 4];
$m_1$ is zero or an integer;
$m_1$ is such that the number-average molecular mass of the polymer of general formula [Chem 3] ranges from 500 g/mol to 50 000 g/mol, preferably from 700 g/mol to 20 000 g/mol;
m is an integer other than zero;
m is such that the number-average molecular mass of the polymer of general formula [Chem 4] ranges from 500 g/mol to 50 000 g/mol, preferably from 700 g/mol to 20 000 g/mol;
p is an integer equal to 0, 1 or 2, p preferably being 0 or 1;
$R^{al}$ represents a divalent hydrocarbon-based radical derived from a diol by replacement of each of the two hydroxyl groups with a free valency or represents the radical $R^2$;
$R^{ac}$ represents a divalent hydrocarbon-based radical derived from a dicarboxylic acid by replacement of each of the two carboxyl groups COOH with a free valency;
$[Y]_q$ represents a repeating unit of general formula [Chem 28]

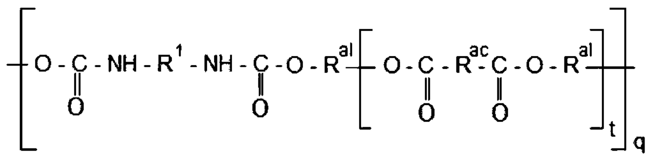

t is a number such that the polyester diol of general formula [Chem 7]

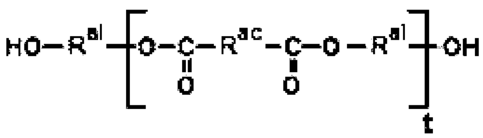

has a hydroxyl number IOH of between 4 and 60 mg KOH/g; q is an integer other than zero;
t and q are such that the number-average molecular mass of the polymer of general formula [Chem 5] is between 400 g/mol and 50 000 g/mol, said hydroxyl number IOH and said average molecular mass being determined according to the standard ISO 14900:2001.

**3.** Composition according to either of the preceding claims, in which the curing catalyst is chosen from the group consisting of amines, organometallic compounds, acids and derivatives thereof, and mixtures thereof.

**4.** Composition according to one of the preceding claims, also comprising a tackifying resin; preferably a tackifying resin chosen from terpene phenolic resins, hydrocarbon resins, rosin resins; acrylic resins and mixtures thereof.

**5.** Composition according to one of the preceding claims, in which the polyvinyl ether compound has a K value of from 40 to 120, and preferably from 40 to 70, said K value being measured according to the standard ISO 1628-1.

**6.** Composition according to any one of the preceding claims, in which the polyvinyl ether compound has a glass transition temperature of from -60 to 0°C, and preferably from -50 to -5°C, said glass transition temperature being measured according to the standard NF EN ISO 11357-2.

**7.** Composition according to one of the preceding claims, in which the polyvinyl ether compound has a content of from 1% to 60% by weight, preferably from 5% to 40% by weight, and preferably from 5% to 25% by weight relative to the total weight of the composition.

**8.** Composition according to one of the preceding claims, also comprising at least one silsesquioxane.

**9.** Composition according to one of the preceding claims, said composition being a one-component composition.

**10.** Composition according to any one of Claims 1 to 9, said composition being a two-component composition comprising:

a part A comprising said at least one silyl polymer; and
a part B comprising said at least one curing catalyst;
said at least one polyvinyl ether compound being present in part A and/or in part B of the composition.

**11.** Use of the composition according to one of the preceding claims as an adhesive.

**12.** Self-adhesive article comprising at least one support layer and at least one layer of the composition according to one of the preceding claims.

**13.** Self-adhesive article according to Claim 12, said article being chosen from dressings, bandages and medical tapes.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2013136108 A1
- WO 2019115952 A1
- WO 2019186014 A1
- WO 03087254 A2
- WO 2020016581 A1
- JP S6389559 A
- JP H0782492 A
- JP 3473049 B
- JP H10147723 A
- EP 2336208 A1
- WO 2009106699 A2
- EP 1829928 A1
- EP 2865694 A1
- EP 2865728 A1
- WO 2008107331 A1


### Littérature non-brevet citée dans la description


- *CHEMICAL ABSTRACTS*, 42856-62-2
- *CHEMICAL ABSTRACTS*, 4457-71-0
- *CHEMICAL ABSTRACTS*, 2157-31-5
- *CHEMICAL ABSTRACTS*, 3121-82-2
- *CHEMICAL ABSTRACTS*, 53120-74-4
- *CHEMICAL ABSTRACTS*, 81554-20-3
- *CHEMICAL ABSTRACTS*, 126-30-7
- *CHEMICAL ABSTRACTS*, 115-76-4
- *CHEMICAL ABSTRACTS*, 78-26-2
- *CHEMICAL ABSTRACTS*, 115-84-4
- *CHEMICAL ABSTRACTS*, 2163-42-0
- *CHEMICAL ABSTRACTS*, 14690-00-7
- *CHEMICAL ABSTRACTS*, 31952-16-6
- *CHEMICAL ABSTRACTS*, 24765-57-9
- *CHEMICAL ABSTRACTS*, 15208-19-2
- *CHEMICAL ABSTRACTS*, 49623-11-2
- *CHEMICAL ABSTRACTS*, 80220-07-1
- *CHEMICAL ABSTRACTS*, 928-40-5
- *CHEMICAL ABSTRACTS*, 4089-71-8
- *CHEMICAL ABSTRACTS*, 82111-97-5
- *CHEMICAL ABSTRACTS*, 23433-04-7
- *CHEMICAL ABSTRACTS*, 1117-86-8
- *CHEMICAL ABSTRACTS*, 23433-05-8
- *CHEMICAL ABSTRACTS*, 27143-31-3
- *CHEMICAL ABSTRACTS*, 109359-36-6
- *CHEMICAL ABSTRACTS*, 75656-41-6
- *CHEMICAL ABSTRACTS*, 3207-95-2
- *CHEMICAL ABSTRACTS*, 76779-60-7
- *CHEMICAL ABSTRACTS*, 85018-58-2
- *CHEMICAL ABSTRACTS*, 42789-13-9
- *CHEMICAL ABSTRACTS*, 40326-00-9
- *CHEMICAL ABSTRACTS*, 13686-96-9
- *CHEMICAL ABSTRACTS*, 85018-64-0
- *CHEMICAL ABSTRACTS*, 85018-65-1
- *CHEMICAL ABSTRACTS*, 85018-63-9
- *CHEMICAL ABSTRACTS*, 35449-36-6
- *CHEMICAL ABSTRACTS*, 48074-20-0
- *CHEMICAL ABSTRACTS*, 128705-94-2
- *CHEMICAL ABSTRACTS*, 112548-49-9
- *CHEMICAL ABSTRACTS*, 109217-58-5
- *CHEMICAL ABSTRACTS*, 48074-21-1
- *CHEMICAL ABSTRACTS*, 85018-66-2
- *CHEMICAL ABSTRACTS*, 35449-37-7
- *CHEMICAL ABSTRACTS*, 13006-29-6
- *CHEMICAL ABSTRACTS*, 1119-87-5
- *CHEMICAL ABSTRACTS*, 33666-71-6
- *CHEMICAL ABSTRACTS*, 85018-68-4
- *CHEMICAL ABSTRACTS*, 85018-69-5
- *CHEMICAL ABSTRACTS*, 85018-70-8
- *CHEMICAL ABSTRACTS*, 5658-47-9
- *CHEMICAL ABSTRACTS*, 80158-99-2
- *CHEMICAL ABSTRACTS*, 62870-49-9
- *CHEMICAL ABSTRACTS*, 38146-95-1
- *CHEMICAL ABSTRACTS*, 39516-24-0
- *CHEMICAL ABSTRACTS*, 39516-27-3
- *CHEMICAL ABSTRACTS*, 22092-59-7
- *CHEMICAL ABSTRACTS*, 20999-41-1
- *CHEMICAL ABSTRACTS*, 91635-53-9
- *CHEMICAL ABSTRACTS*, 107-21-1
- **W. WAGNER et al.** *International Steam Tables - Properties of Water and Steam based on the Industrial Formulation IAPWS-IF97*